# EUROPEAN PATENT SPECIFICATION

(11) **EP 0 831 829 B1**
(45) Date of publication and mention of the grant of the patent: **20.08.2003**
(21) Application number: 95924027.6
(22) Date of filing: 07.06.1995
(51) Int. Cl.: A61K 31/505, A61K 31/52, C07D 239/00, C07D 209/00, C07D 487/04, C07D 207/34

(54) **HETEROCYCLIC RING-FUSED PYRIMIDINE DERIVATIVES**
HETEROCYCLISCHE KONDENSIERTE PYRIMIDIN-DERIVATE
DERIVES DE PYRIMIDINE HETEROCYCLIQUES A NOYAUX CONDENSES

(43) Date of publication of application: 01.04.1998
(73) Proprietor: PFIZER INC., New York, N.Y. 10017 (US)
(72) Inventor: ARNOLD, Lee, D., Quaker Hill, CT 06375 (US); MOYER, Mikel, P., Clinton, CT 06413 (US); SOBOLOV-JAYNES, Susan, B., Ivoryton, CT 06442 (US)
(74) Representative: Atkinson, Jonathan David Mark
(86) International application number: US9507881
(87) International publication number: WO96040142

(56) References cited:
- EP-A- 0 075 881
- EP-A- 0 089 055
- EP-A- 0 160 910
- EP-A- 0 260 491
- WO-A-94/13676
- WO-A-95/01355
- US-A- 4 012 513
- US-A- 5 106 974
- US-A- 5 378 700

## Description

### Background of the Invention

This invention relates to heterocyclic ring-fused pyrimidine derivatives and methods of using the same In the treatment of hyperproliferative diseases, such as cancers and acnes, in mammals.

Many of the current treatment regimes for cancer utilize compounds which inhibit DNA synthesis. Such compounds are toxic to cells generally but their toxic effect on the rapidly dividing tumor cells can be beneficial. Alternative approaches to anti-cancer agents which act by mechanisms other than the inhibition of DNA synthesis have been explored In order to enhance the selectivity of action against cancer cells.

It is known that a cell may become cancerous by virtue of the transformation of a portion of its DNA Into an oncogene (i.e. a gene which, on activation, leads to the formation of malignant tumor cells). Many oncogenes encode proteins which are aberrant tyrosine kinases capable of causing cell transformation. Alternatively, the overexpression of a normal proto-oncogenic tyrosine kinase may also result in proliferative disorders, sometimes resulting in a malignant phenotype.

Receptor tyrosine kinases are large enzymes which span the cell membrane and possess an extracellular binding domain for growth factors such as epidermal growth factor, a transmembrane domain, and an intracellular portion which functions as a kinase to phosphorylate specific tyrosine residues in proteins and hence to influence cell proliferation. It is known that such kinases are frequently aberrantly expressed in common human cancers such as breast cancer, gastrointestinal cancer such as colon, rectal or stomach cancer, leukemia, and ovarian, bronchial or pancreatic cancer. It has also been,shown that epidermal growth factor receptor (EGFR) which possesses tyrosine kinase activity is mutated and/or overexpressed in many human cancers such as brain, lung, squamous cell, bladder, gastric, breast, head and neck, oesophageal, gynecologlcal and thyroid tumors.

Accordingly, it has been recognized that inhibitors of receptor tyrosine kinases are useful as a selective inhibitors of the growth of mammalian cancer cells. For example, erbstatin, a tyrosine kinase inhibitor selectively attenuates the growth in athymic nude mice of a transplanted human mammary carcinoma which expresses epidermal growth factor receptor tyrosine kinase (EGFR) but is without effect on the growth of another carcinoma which does not express the EGF receptor.

International Patent Publication WO95/01355 describes certain 9-deazahypoxanthines as purine nucleocide phosphorylase (PNP) inhibitors.

EP0260491 describes certain 9-deazaguanes which can have activity as PNP inhibitors.

EP0089055 discloses certain 7-deazapurine derivatives and methods of producing the same.

EP0075881 also discloses certain 7-deazapurine derivatives.

EP0160910 discloses further 7-deazapurine derivatives and a method of making these compounds.

WO94/13676 discloses certain pyrrolopyridines as CRF antagonists.

Various other compounds, such as styrene derivatives, have also been shown to possess tyrosine kinase inhibitory properties. More recently three European patent publications, namely EP 0 566 226 A1, EP 0 602 851 A1 and EP 0 520 722 A1 have disclosed that certain heteroaryl-fused pyrimidine derivatives possess anti-cancer properties which result from their tyrosine kinase inhibitory properties. Also PCT publication WO 92/20642 discloses bis-mono and bicyclic aryl and heteroaryl compounds as tyrosine kinase inhibitors.

European patent publication EP 0 496 617 A1 discloses certain pyrazolo[3,4-d]pyrimidines and pyrrolo[2,3-d]pyrimidines which possess adenosine kinase inhibitory properties.

European patent publication EP 0 475 413 A2 discloses certain carbocyclic nucleoside analogs as useful immunosuppressants.

European patent publication EP 0 414 386 A1 discloses certain pyrido[2,3-d]pyrimidines as fungicides, insecticides and miticides. The synthesis and antiallergic activity of 9-aryl-8-azaadenine derivatives is described in II Farmco -Ed. Sc., vol 35, fasc. 4 p308-323 (1980).

Co-pending United States patent applications (United States Serial Nos. 08/200,359 (WO 95/23141) and 08/413,300 (WO 96/30347) and PCT application docket no. PC8836A (WO 96/30347) assigned to the Assignee of this application, describe optionally substituted indolyl- and phenylamino quinazolines, respectively, which are useful in the treatment of hyperproliferative diseases involving receptor tyrosine kinases. In addition U.S. Patent No. 4.012,513 discloses certain 1-(heterocyclic)-indol-3-yl-acetic acid derivatives that have antiinflammatory, analgesic and antipyretic activity.

Although the anti-cancer compounds described above make a significant contribution to the art there is a continuing search in this field of art for improved anti-cancer pharmaceuticals.

### Summary of the Invention

This invention is directed to compounds of the Formula 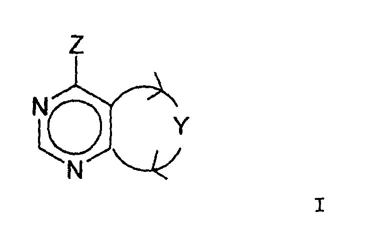 and stereoisomers, and pharmaceutically acceptable salts thereof, wherein Y, in the direction shown by the arrow in formula I, is selected from CR³= CR³-NR⁴- and -NR⁴-CR³=CR³-;
Z is NR¹R² wherein R¹ is H and R² is phenyl substituted by (R⁵)ₘ or Q or R¹R²N is a group of the formula 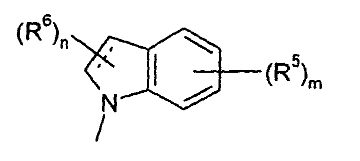 wherein the dotted line represents an optional double bond;
each R³ is attached to a carbon atom in Y and is independently selected from
a. hydrogen, trifluoromethyl, halo, nitro, hydroxy, amino, cyano, (C₁-C₄)alkyl, (C₁-C₄)alkoxy, (C₁-C₄)alkoxycarbonyl, (C₁-C₄)alkanoyloxy, (C₁-C₄)alkanoylamino, carboxy, phenoxy, benzoyloxy, carbamoyl, mono-N-or di-N-N-di-(C₁-C₄)alkylcarbamoyl, mono-N- or di-N,N-(C₁-C₄)alkylamino, mono-N- or di-N,N-(hydroxy(C₂-C₄)alkyl)amino, mono-N-or di-N,N-((C₁-C₄)alkoxy(C₂-C₄)alkyl)amino, or such groups substituted on (C₁-C₄)alkyl;
b. hydroxy(C₂-C₄)alkoxy(C₁-C₄)alkyl, (C₁-C₄)alkoxy-(C₁-C₄)alkoxy-(C₁-C₄)alkyl, hydroxy(C₂-C₄)alkylthio(C₁-C₄), (C₁-C₄)alkoxy(C₂-C₄)alkylthio(C₁-C₄)alkyl, hydroxyamino, benzoylamino, mono-N- or di-N,N-(C₁-C₄)alkylcarbamoylmethylamino, carbamoylmethylamino, (C₁-C₄)alkoxycarbonylamino, (C₁-C₄)alkanoylamino, carboxymethylamino, (C₁-C₄)alkoxycarbonylmethylamino, (C₁-C₄)alkoxyamino, (C₂-C₄)alkanoyloxyamino, (C₁-C₄)alkylsulfonylamino, ureido,(C₁-C₄)alkoxy (C₁-C₄)alkylcarbonylamino,(C₁-C₄)alkylsulfinyl,(C₁-C₄)alkylsulfonyl, (C₁-C₄)alkoxy(C₂-C₄)alkylthio, mono-, di- or trifluoromethyloxy, (C₁-C₄)alkylenedioxy, guanidino, aminocarbonyl, mono-N- or di-N,N-(C₁-C₄)alkylaminocarbonyl, carboxymethoxy, (C₁-C₄)alkoxycarbonylmethoxy, carbamoylmethoxy, mono-N or di-N,N-(C₁-C₄)alkylcarbamoylmethoxy, mono-N- or di-N,N-(hydroxy(C₂-C₄)alkyl)carboxamido, mon-N- or di-N,N-((C₁-C₄)alkoxy (C₂-C₄)alkyl)carboxamido or bis((C₁-C₄)alkanesulfonyl)amido; or
c. (C₂-C₄)alkoxy, (C₂-C₄)alkylthio, (C₂-C₄)alkanoyloxy, (C₂-C₄)alkylamino, (C₁-C₄)alkyl(C₁-C₄)alkylenedioxy, (C₂-C₄)alkanoylamino, (C₂-C₄)alkenyl, or (C₂-C₄)alkynyl; each such group substituted with amino, halo, hydroxy, (C₂-C₄)alkanoyloxy, (C₁-C₄)alkoxy, mono-N- or di-N,N-(C₁-C₄)alkylamino, mono-N or di-N,N-(hydroxy(C₂-C₄)alkyl)amino, mono-N or di-N,N-((C₁-C₄)alkoxy(C₂-C₄)alkoxy(C₂-C₄)alkyl)amino, (C₁-C₄)alkanoylamino, carboxy(C₁-₄)alkylthio(C₄)alkoxy, carboxy, (C₁-C₄)alkoxycarbonyl, carbamoyl, mono-N or di-N,N-(C₁-C₄)alkylcarbamoyl, carboxamido, mono-N- or di-N,N-(C₁-C₄)alkylcarboxamido or mono-N- or di-N,N-(hydroxy(C₂-C₄)alkyl)carboxamido; and any phenyl in an R³ substituent is optionally mono- or di- substituted with halo, nitro, trifluoromethyl, hydroxy, (C₁-C₄)alkoxy, (C₁-C₄)alkyl, amino, mono-N-alkylamino, or N,N-dialkylamino;
R⁴ is attached to the N-atom in Y and is selected from: hydrogen, (C₁-C₄)alkyl, (C₁-C₄)alkoxycarbonyl, (C₁-C₄)alkanoyl, (C₁-C₄)alkylsulfonyl, arylsulfonyl, allyl; or a (C₂-C₄)alkyl, (C₂-C₄)alkanoyl, or C₂-C₄)alkoxycarbonyl, (C₂-C₄)alkylsulfonyl, each such group substituted with amino, halo, hydroxy, (C₂-C₄)alkanoyloxy, (C₁-C₄)alkoxy, mono-N-or di-N,N-(C₁-C₄)alkylamino, mono-N or di-N,N-(hydroxy (C₂-C₄)alkylamino, mono-N or di-N,N-((C₁-C₄)alkoxy(C₂-C₄)alkyl)amino, (C₁-C₄)alkanoylamino, carboxy, (C₁-C₄)alkoxycarbonyl, carbamoyl, mono-N- or di-N,N-(C₁-C₄)alkylcarbamoyl, carboxamido, mono-N- or di-N,N-(C₁-C₄)alkylcarboxamido or mono-N- or di-N,N-(hydroxy(C₂-C₄)alkyl)carboxamido;
each R⁵ is independently selected from mono-, di- or tri-fluoromethyl, halo, nitro, hydroxy, amino, azido, isothiocyano, (C₁-C₄)alkyl, (C₁-C₄)alkoxy, (C₂-C₆)alkenyl, (C₂-C₆)alkynyl, (C₁-C₄)alkylenedioxy, cyano, trifluoromethylcarbonylamino, (C₁-C₄)alkanoylamino, (C₁-C₄)alkanoyl,N-mono- or N,N-di-(C₁-C₄)alkylamino, (C₁-C₄)alkylsulfonylamino, trifluoromethylsulfonylamino, (C₁-C₄)alkylthio, (C₁-C₄)alkylsulfinyl or (C₁-C₄)alkylsulfonyl, and said (C₁-C₄)alkylenedioxy is linked at both ends to adjacent carbons on the benzene moiety;
each R⁶ is independently selected from hydroxy, amino, N-mono- or N,N-di(C₁-C₄)alkylamino, sulfo, or (C₁-C₄)alkoxy (provided that such groups are not attached to a ring carbon which is directly adjacent to the ring N-), or R⁶ for each occurrence is independently carboxy, hydroxy(C₁-C₄)alkyl, (C₁-C₄)alkoxy(C₁-C₄)alkyl, amino(C₁-C₄)alkyl, mono-N- or di-N,N-(C₁-C₄)alkylamino(C₁-C₄)alkyl, morpholino (C₁-C₄)alkyl, 4-(C₁-C₄)alkyl-piperazin-1-yl(C₁-C₄)alkyl, carboxy(C₁-C₄)alkyl, (C₁-C₄)alkoxycarbonyl, sulfo(C₁-C₄)alkyl, pyridyl(C₁-C₄)alkyl or C₁-C₄)alkyl;
m is an interger from 1 to 3;
n is 0, 1 or 2;
p is 0 or an integer from 1-3;
Q is a 9- or 10-membered bicyclic heteroaryl cyclic moiety, or a hydrogenated derivative thereof, containing one or two nitrogen heteroatoms and optionally containing a further heteroatom selected from nitrogen, oxygen and sulphur, or Q is a 9- or 10-membered bicyclic aryl moiety, or a hydrogenated derivative thereof, which heterocyclic or aryl moiety, or hydrogenated derivatives thereof, may optionally bear one or two substituents selected from halogeno, hydroxy, oxo, amino, nitro, carbamoyl, (C₁-C₄)alkyl, (C₁-C₄)alkoxy, (C₁-C₄)alkylamino, di-[(C₁-C₄)alkyl]amino, (C₂-C₄)alkanoylamino, and (C₂-C₄)alkynyl with the proviso that when Y, in the direction shown by the arrow in formula I, is -NR⁴-CR³=CR³-, R³ = CH₃ and R⁴=H, then R⁵ is not 4-CH₃, 3,5-(CH₃)₂, 2,6-(CH₃)₂, 2-C₂H₅, 4-C₂H₅, 4-n-C₄H₉, 2-Cl, 4-Cl, 3,4-Cl₂, 2-F, or 3-CF₃.

Another aspect of the invention provides a compound as described above wherein each R³ is independently selected from hydrogen, hydroxy, (C₁-C₄)alkoxy, hydroxy(C₂-C₄)alkoxy, amino(C₂-C₄)alkyl, amino(C₂-C₄)alkoxy, (C₁-C₄)alkoxy(C₂-C₄)alkoxy, hydroxy(C₁-C₄)alkyl(C₁-C₄)alkylenedioxy, (C₁-C₄)alkoxy(C₁-C₄)alkyl(C₁-C₄)alkylenedioxy, mono-N- or di-N,N-(C₁-C₄)alkylamino(C₂-C₄)alkoxyl, 3- or 4-(C₁-C₄)alkoxy-(2-hydroxy)-(C₃-C₄)alkoxy, carboxy(C₁-C₄)alkoxy, morpholino(C₂-C₄)alkoxy, imidazol-1-yl(C₂-C₄)alkoxy, 4(C₁-C₄)alkylpiperazin-1-yl-(C₂-C₄)alkoxy, (C₁-C₄)alkoxy(C₁-C₄)alkanoyloxy, nitro, hydroxylamino, amino, phenyl, pyridyl, pyrrolo, imidazolo, thiazolo, benzimidazolo, pyridonyl, mono-N- or di-N,N-(C₁-C₄)alkylamino, (C,-C₄)alkanoylamino, hydroxy(C₂-C₄)alkylamino, (C₁-C₄)alkoxy(C₂-C₄)alkylamino, (C₁-C₄)alkylsulfonamido, morpholino, (C₁-C₄)alkyl-piperazin-1-yl, bis(C₁-C₄)alkanesulfonamido, di-N,N-(C₁-C₄)alkylamino(C₂-C₄)alkylamino, (C₁-C₄)alkylamino(C₂-C₄)alkylamino, piperidin-1-yl, imidazol-1-yl, pyrrolidin-1-yl, (C₁-C₄)alkoxy(C₁-C₄)alkylcarbonylamino, carboxy, (C₁-C₄)alkoxycarbonyl, (C₁-C₄)alkoxycarbonyl(C₁-C₄)alkoxy, amido, mono-N- or di-N,N-(C₁-C₄)alkylaminocarbonyl, mono-N- or di-N,N-(hydroxy(C₂-C₄)alkyl)aminocarbonyl, (C₁-C₄)alkyl, hydroxy(C₁-C₄)alkyl, mono-N- or di-N,N-((C₁-C₄)alkoxy(C₁-C₄)alkyl)amino(C₁-C₄)alkyl, mono-N- or di-N,N-(C₁-C₄)alkylamino(C₁-C₄)alkyl, (C₁-C₄)alkanoylamino(C₁-C₄)alkyl, (C₁-C₄)alkoxy(C₂-C₄)alkoxy(C₁-C₄)alkyl, (C₁-C₄)alkylthio, (C₁-C₄)alkoxy(C₂-C₄)alkylthio or hydroxy(C₂-C₄)alkylthio; and R⁴ is selected from hydrogen, benzyl, phenyl, a (C₂-C₄)alkyl, hydroxy(C₂-C₄)alkyl, or hydroxy(C₂-C₄)alkyl, amino(C₂-C₄)alkyl, (C₂-C₄)alkoxycarbonyl each such group substituted with amino, halo, hydroxy, (C₂-C₄)alkanoyloxy, (C₁-C₄)alkoxy, mono-N- or di-N,N-(C₁-C₄)alkylamino, mono-N or di-N,N-(hydroxy(C₂-C₄)alkyl)amino, mono-N or di-N,N-((C₁-C₄)alkoxy(C₂-C₄)alkyl)amino, sulfonylaryl(C₁-C₄)alkylamine, (C₁-C₄)alkanoylamino, imidazol-1-yl, piperidino, morpholino, piperazin-1-yl-, 4-(C₁-C₄)alkylpiperazin-1-yl-, pyridyl, pyrrolo, imidazolo, thiazolo, pyridonyl, carboxy, (C₁-C₄)alkoxycarbonyl, carbamoyl, mono-N- or di-N,N-(C₁-C₄)alkylcarbamoyl, carboxamido, mono-N- or di-N,N-(C₁-C₄)alkylcarboxamido or mono-N- or di-N,N-(hydroxy(C₂-C₄)alkyl)carboxamido.

Yet another aspect of the invention provides a compound as described above wherein Y, in the direction shown by the arrow in formula I, is -CR³=CR³-NR⁴- and R⁴ is hydrogen.

Another aspect of the invention provides a compound as described above wherein R¹R²N is 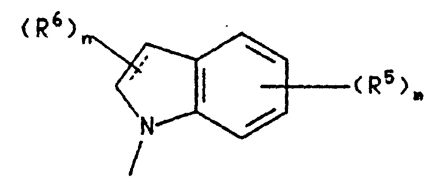 and R⁵, R⁶, m and n are as defined above.

According to another aspect of the invention there is provided a compound as described above wherein each R⁵ is independently selected from 4-hydroxy, 4-amino, 5-fluoro, 5-hydroxy, 5-amino; 6-halo, 6-methyl, 6-ethenyl, 6-ethynyl, 6-nitro and 7-methyl and each R⁶ is independently selected from hydroxy, amino, N-mono- or N,N-di-(C₁-C₄)alkylamino, sulfo, or (C₁-C₄)alkoxy (provided that such groups are not attached to a ring carbon which is directly adjacent to the ring N-), or R⁸ for each occurrence is independently carboxy, hydroxy(C₁-C₄)alkyl, (C₁-C₄)alkoxy(C₁-C₄)alkyl, amino(C₁-C₄)alkyl, mono-N- or di-N,N-(C₁-C₄)alkylamino(C₁-C₄)alkyl, morpholino (C₁-C₄)alkyl, 4-(C₁-C₄)alkyl-piperazin-1-yl(C₁-C₄)alkyl, carboxy(C₁-C₄)alkyl, (C₁-C₄)alkoxycarbonyl, sulfo(C₁-C₄)alkyl, pyridyl(C₁-C₄)alkyl and (C₁-C₄)alkyl.

Another aspect of the invention provides a compound as described above wherein R¹ is H and R² is (R⁵)ₘ-substituted phenyl wherein R⁵ and m are as defined above.

Yet another aspect of the invention provides a compound as described above wherein each R⁵ is independently selected from 4-fluoro-3-chloro, 3-trifluoromethyl, 4-fluoro-3-trifluoromethyl, 3-nitro-4-chloro, 3-nitro-4-fluoro, 4-fluoro-3-bromo, 3-iodo-5-amino, 3-methyl-4-fluoro, 4-amino, 3-fluoro, 3-hydroxy, 3-amino, 3-halo, 3-methyl, 3-ethenyl, 3-ethynyl, 3-nitro and 4-methyl.

According to another aspect of the invention there is provided a compound as described above wherein R¹ is H and R² is Q.

Another aspect of the invention provides a compound as described above wherein Q is selected from pyrrolo 1,2,3,5-tetrahydro-pyrrolo[2,3-f]indole, 4-, 5-, 6-indolyl, 1H-benzimidazol-4-yl, 1H-benzimidazol-5-yl, 1H-indazol-4-yl, 1H-indazol-5-yl, 1H-indazol-6-yl, 1H-indazol-7-yl, 1H-benzotrizol-4-yl, 1H-benzotrizol-5-yl, 1H-benzotrizol-6-yl, 5- or 6-benzoxazolyl, 5- or 6-benzothiazolyl, benzo[c][2,1,3]thiadiazol-4-yl, 2-, 3-, 4-, 5-, 6-, 7- or 8-quinolyl, 1-, 3-, 4-, 5-, 6-, 7- or 8-isoquinolyl, 4-, 5-, 6-, 7- or 8-cinnolinyl, 5-, 6-, 7- or 8-quinazolinyl, or 2-, 5-, or 6-quinoxalinyl, which may optionally bear one or two substituents selected from fluoro, bromo, chloro,methyl, ethyl, ethenyl, ethynyl and methoxy.

Yet another aspect of the invention provides a compound as described above wherein Q is selected from pyrrolo 5-indolyl, 1H-indazol-5-yl, 1H-benzotriazol-5-yl, 6-benzothiazolyl, benzo[c][2,1,3]thiadiazol-4-yl, 5-quinolyl, 6-quinolyl, 8-quinolyl, 5-isoquinolyl, or 5-quinoxalinyl, which may optionally bear one or two substituents selected from fluoro, bromo, chloro,methyl, ethyl, ethenyl, ethynyl and methoxy.

Preferred compounds of formula I are selected from the group consisting of
(3-ethynyl-phenyl)-(7H-pyrrolo[2,3-d]pyrimidin-4-yl)-amine hydrochloride;
(3-chloro-phenyl)-(7H-pyrrolo[2,3-d]pyrimidin-4-yl)-amine hydrochloride;
4-(6-chloro-2,3-dihydro-indol-1-yl)-7H-pyrrolo[2,3-d]pyrimidine hydrochloride;
(7H-pyrrolo[2,3-d]pyrimidin-4-yl)-m-tolyl-amine hydrochloride;
(1H-indol-5-yl)-(7H-pyrrolo[2,3-d]pyrimidin-4-yl)-amine hydrochloride;
(6-methylindolin-1-yl)-(7H-pyrrolo[2,3-d]pyrimidin-4-yl)-amine;
(benzo[b]thien-5-yl)-(7H-pyrrolo[2,3-d]pyrimidin-4-yl)-amine;
(6-chloro-5-fluoroindolin-1-yl)-(7H-pyrrolo[2,3-d]pyrimidin-4-yl)-amine;
(1H-indazol-5-yl)-(7H-pyrrolo[2,3-d]pyrimidin-4-yl)-amine;
1-(4-m-tolylamino-pyrrolo[2,3-d]pyrimidin-7-yl)-ethanone hydrochloride;
(5-iodo-7H-pyrrolo[2,3-d]pyrimidin-4-yl)-m-tolyl-amine;
(7-benzenesulfonyl-7H-pyrrolo[2,3-d]pyrimidin-4-yl)-(3-ethynyl-phenyl)-amine;
4-(6-chloro-2,3-dihydro-indol-1-yl)-5H-pyrrolo[3,2-d]pyrimidin-6-ol;
(3-ethynyl-phenyl)-[7-(2-morpholin-4-yl-ethyl)-7H-pyrrolo[2,3-d]pyrimidin-4-yl]-amine;
(3-ethynyl-phenyl)-[7-(2-methoxy-ethyl)-7H-pyrrolo[2,3-d]pyrimidin-4-yl]-amine;
(3-ethynyl-phenyl)-{7-[2-(2-methoxy-ethoxy)-ethyl]-7H-pyrrolo[2,3-d]pyrimidin-4-yl}-amine;
(7-allyl-pyrrolo[2,3-d]pyrimidin-4-yl)-(3-ethynyl-phenyl)-amine hydrochloride;
(3-ethynyl-phenyl)-(7-methyl-pyrrolo[2,3-d]pyrimidin-4-yl)-amine hydrochloride;
(5-bromo-7H-pyrrolo[2,3-d]pyrimidin-4-yl)-(3-ethynyl-phenyl)-amine;
(3-ethynyl-phenyl)-(5-iodo-7H-pyrrolo[2,3-d]pyrimidin-4-yl)-amine;
4-(3-ethynyl-phenylamino)-7H-pyrrolo[2,3-d]pyrimidine-5-carboxylic acid;
(3-ethynyl-phenyl)-(5-methyl-7H-pyrrolo[2,3-d]pyrimidin-4-yl)-amine hydrochloride;
N-(5-iodo-7H-pyrrolo[2,3-d]pyrimidin-4-yl)-N-m-tolyl-acetamide;
4-(3-ethynyl-phenylamino)-7H-pyrrolo[2,3-d]pyrimidine-5-carboxylic acid methyl esterhydrochloride;(3-ethynyl-phenyl)-(5-cyano-7H-pyrrolo[2,3-d]pyrimidin-4-yl)-amine;
(3-ethynyl-phenyl)-(5-methylsulfanyl-7H-pyrrolo[2,3-d]pyrimidin-4-yl)-amine.

Most preferred compounds of the formula I as described above are selected from
(3-ethynyl-phenyl)-(7H-pyrrolo[2,3-d]pyrimidin-4-yl)-amine;
(3-chloro-phenyl)-(7H-pyrrolo[2,3-d]pyrimidin-4-yl)-amine;
(3-ethynyl-phenyl)-(5-methyl-7H-pyrrolo[2,3-d]pyrimidin-4-yl)-amine;
4-(3-ethynyl-phenylamino)-7H-pyrrolo[2,3-d]pyrimidine-5-carboxylic acid methyl ester;
4-(3-ethynyl-phenylamino)-7H-pyrrolo[2,3-d]pyrimidine-5-carbonitrile;
(3-ethynyl-phenyl)-(5-methylsulfanyl-7H-pyrrolo[2,3-d]pyrimidin-4-yl)-amine;
(1H-indol-5-yl)-(7H-pyrrolo[2,3-d]pyrimidin-4-yl)-amine;
(5-bromo-7H-pyrrolo[2,3-d]pyrimidin-4-yl)-(3-ethynyl-phenyl)-amine;

The invention also provides the use of a compound of formula I for the preparation of a medicament for treating hyperproliferative disorders

According to an embodiment of the invention the hyperproliferative disorder is cancer.

In another embodiment of the invention the disorder is brain, lung, squamous cell, bladder, gastric, pancreatic, hepatic, renal, colorectal, breast, head, neck, oesophageal, gynecological or thyroid cancer.

In another embodiment of the invention the hyperproliferative disorder is noncancerous.

In another embodiment of the invention the noncancerous hyperproliferative disorder is psoriasis or benign prostatic hyperplasia.

The invention further provides a pharmaceutical composition for the treatment of hyperproliferative disorder in a mammal which comprises a hyperproliferative disease treating amount of a compound of formula I and a pharmaceutically acceptable carrier.

In the present application certain terms are defined as follows:

By halo is meant chloro, bromo, lodo, or fluoro.

By alkyl is meant straight chain or, when C₃ or larger, a cyclic or, when C₂ or larger, a branched saturated hydrocarbon.

As used herein, the expression "reaction-inert solvent" refers to a solvent which does not interact with starting materials, reagents, intermediates or products in a manner which adversely affects the yield of the desired product.

Other features and advantages will be apparent from the specification and claims which describe the invention.

### Detailed Description of the Invention

The Formula I compounds, or pharmaceutically acceptable salts thereof can be prepared by any process known to be applicable to the preparation of chemically-related compounds.

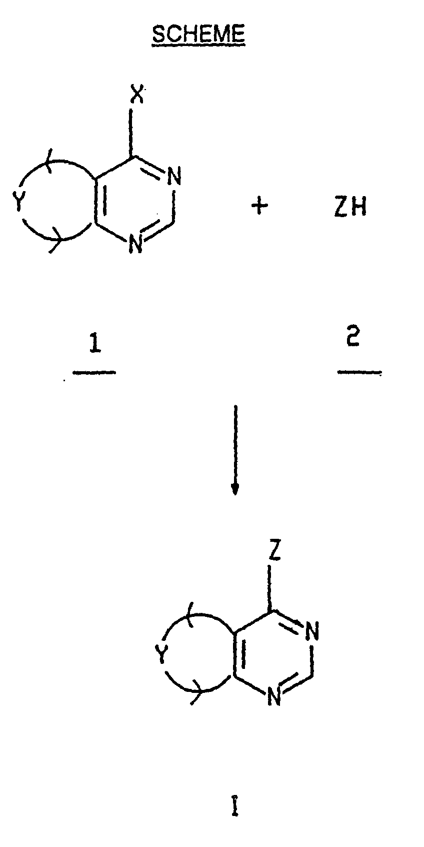

As shown in the Scheme the Formula I compounds can, generally, be made prepared from the 4-chloro or hydroxy derivatives of the appropriately substituted heteroaryl-fused pyrimidine 1 using the appropriately substituted amine ZH 2.

Typically the appropriately substituted 4-haloheteroaryl-fused pyrimidine 1 (or a heteroaryl-fused pyrimidine bearing a suitable displaceable leaving group in the 4-position such as aryloxy, alkyl sulfinyloxy such as trifluoromethanesulfonyloxy, arylsulfinyloxy, siloxy, cyano, pyrazolo, triazolo or tetrazolo), preferably a haloheteroaryl such as 4-chloroheteroaryl derivative, is reacted with the appropriate amine 2 In a solvent such as a (C₁-C₈)alcohol, dimethylformamide (DMF), N-methylpyrrolidin-2-one, chloroform, acetonitrile, tetrahydrofuran (THF), dimethylsulfoxide (DMSO), 1-4 dioxane, pyridine or other aprotic solvent. The combination can be effected in the presence of a base, preferably an alkali or alkaline earth metal carbonate or hydroxide or a tertiary amine base, such as pyridine, 2,6-lutidine, collidine, N-methyl-morpholine, triethylamine, diethyl isopropyl amine, 4-dimethylamino-pyridine or N,N-dimethylaniline. These bases are hereinafter refered to as "suitable bases". The mixture is maintained at a temperature of from ambient to reflux, preferably from about 35°C to reflux, until substantially no remaining 4-haloheteroaryl-fused pyrimidine can be detected, typically about 2 hours to about 72 hours. The reaction is preferably performed under an inert atmosphere such as dry nitrogen gas.

Generally, the reactants are combined stoichiometrically when a suitable amine base is used, although, for those compounds where a salt (typically the HCl salt) of the amine is used, it is preferable to use excess amine 2, generally an extra equivalent of the amine 2. Alternatively, if an amine base is not used an excess of the amine reactant may be used)

For those compounds where a sterically hindered amine (such as a 2-alkylindoline) or very reactive 4-haloheteroaryl-fused pyrimidine is used it is preferable to use t-butyl alcohol or a polar aprotic solvent such as dimethylformamide or N-methylpyrrolidin-2-one as the solvent.

Other Formula I compounds may be prepared by the following appropriate reactions subsequent to the above coupling.

Compounds of Formula I wherein R³ or R⁵ is a primary amino or hydroxyamino may be prepared by the reduction of Formula I compounds wherein R³ or R⁵ is a nitro group.

The reduction may conveniently be carried out by any of the many procedures known for such transformations. The reduction may be carried out, for example, by the hydrogenation of a solution of the nitro compound in a reaction-inert solvent in the presence of a suitable metal catalyst such as palladium or platinum. A further suitable reducing agent is, for example, an activated metal such as activated iron (produced by washing iron powder with a dilute solution of an acid such as hydrochloric acid). Thus, for example, the reduction may be carried out by heating a mixture of the nitro compound and the activated metal in a solvent such as a mixture of water and an alcohol, for example, methanol or ethanol, to a temperature in the range, for example, 50 to 150°C, conveniently at or near 70°C.

For the production of those compounds of Formula I wherein R⁵ or R⁶ incorporates a primary or secondary amino moiety (other than the amino group intended to react with the quinazoline), such free amine is preferably protected prior to the above described reaction followed by deprotection, subsequent to the above described reaction with 4-haloquinazoline.

For a description of protecting groups and their use, see T.W. Greene and P.G.M. Wuts, "Protective Groups In Organic Synthesis" Second Ed., John Wiley & Sons, New York, 1991.

Nitrogen protecting groups are well known in the art, including (C₁-C₆)alkoxycarbonyl, optionally substituted benzyloxycarbonyl, aryloxycarbonyl, trityl, vinyloxycarbonyl, O-nitrophenylsulfonyl, diphenyiphosphinyl, p-toluenesulfonyl, and benzyl. The addition of the nitrogen protecting group may be carried out in a chlorinated hydrocarbon solvent such as methylene chloride or 1,2-dichloroethane, or an ethereal solvent such as glyme, diglyme or THF, in the presence or absence of a tertiary amine base such as triethylamine, diisopropylethylamine or pyridine, preferably triethylamine, at a temperature from about 0°C to about 50°C, preferably about ambient temperature. Alternatively, the protecting groups are conveniently attached using Schotten-Baumann conditions.

Subsequent to the above described amine coupling reaction the protecting group may be removed by deprotecting methods known to those skilled in the art such as trifluoroacetic acid in methylene chloride for the tert-butoxycarbonyl protected products.

Compounds of the Formula I wherein R³ is hydroxy, may preferably be prepared by cleavage of a Formula I compound wherein R³ is (C₁-C₄)alkoxy.

The cleavage reaction may conveniently be carried out by any of the many procedures known for such a transformation. Treatment of the heteroaryl-fused pyrimidine derivative of Formula I with molten pyridine hydrochloride (20-30 eq.) at 150 to 175°C may be employed for O-dealkylations. Alternatively, the reaction may be carried out, for example, by treatment of the heteroaryl-fused pyrimidine derivative with an alkali metal (C₁-C₄)alkylsulphide such as sodium ethanethiolate or, for example, by treatment with an alkali metal diarylphosphide such as lithium diphenylphosphide. Alternatively the cleavage reaction may conveniently be carried out, for example, by treatment of the heteroaryl-fused pyrimidine derivative with a boron or aluminum trihalide such as boron tribromide. Such reactions are preferably carried out in the presence of a reaction-inert solvent and at a suitable temperature.

For the production of those compounds of Formula I wherein R³ Is a (C₁-C₄)alkylsulphinyl or (C₁-C₄)alkylsulphony group, the oxidation of a Formula I compound wherein R³ is a (C₁-C₄)alkylthio group Is preferred.

A suitable oxidizing agent is, for example, an agent known in the art for the oxidation of thio to sulphinyl and/or sulphonyl, for example, hydrogen peroxide, a peracid (such as 3-chloroperoxybenzoic or peroxyacetic acid), an alkali metal peroxysulphate (such as potassium peroxymonosulphate), chromium trioxide or gaseous oxygen in the presence of platinum. The oxidation is generally carried out under as mild conditions as possible and with the required stoichiometric amount of oxidizing agent in order to reduce the risk of over oxidation and damage to other functional groups. In general the reaction is carried out in a suitable solvent such as methylene chloride, chloroform, acetone, tetrahydrofuran or tert-butyl methyl ether and at a temperature, for example, -25 to 50°C, conveniently at or near ambient temperature, that is In the range of 15 to 35°C. When a compound carrying a sulphinyl group Is required a milder oxidizing agent may also be used, for example sodium or potassium metaperiodate, conveniently in a polar solvent such as acetic acid or ethanol. It will be appreciated that when a compound of the Formula I containing a (C₁-C₄)alkylsulphonyl group is required, it may be obtained by oxidation of the corresponding (C₁-C₄)alkylsulphinyl compound as well as of the corresponding (C₁-C₄)alkylthio compound.

For the production of those compounds of Formula I wherein R³ is (C₂-C₄)alkanoylamino or substituted (C₂-C₄)alkanoylamino, ureido, 3-phenylureido, benzamido, or sulfonamido, the acylation or sulfonylation of a Formula I compound wherein R³ is amino is appropriate.

A suitable acylating agent is, for example, any agent known in the art for the acylation of amino to acylamino, for example an acyl halide (e.g., a (C₂-C₄)alkanoyl chloride or bromide or a benzoyl chloride or bromide), an alkanoic acid anhydride or mixed anhydride (e.g., (C₂-C₄)alkanoic acid anhydride such as acetic anhydride or the mixed anhydride formed by the reaction of an alkanoic acid and a (C,-C₄)alkoxycarbonyl halide, for example (C₁-C₄)alkoxycarbonyl chloride, in the presence of a suitable base). For the production of those compounds of Formula I wherein R' is ureido or 3-phenylureido, a suitable acylating agent Is, for example, a cyanate, for example an alkali metal cyanate such as sodium cyanate or, for example, an isocyanate such as phenyl isocyanate. N-sulfonylations may be carried out with suitable sulfonyl halides or sulfonylanhydrides in the presence of a tertiary amine base. In general the acylation or sulfonylation is carried out in a reaction-inert solvent and at a temperature, in the range, for example, -30 to 120°C, conveniently at or near ambient temperature.

For the production of those compounds of Formula I wherein R³ is (C₁-C₄)alkoxy or substituted (C₁-C₄)alkoxy or R¹ is (C₁-C₄)alkylamino or substituted mono-N- or di-N,N-(C₁-C₄)alkylamino, the alkylation, preferably in the presence of a suitable base, of a Formula I compound wherein R¹ is hydroxy or amino, as appropriate, is preferred.

A suitable alkylating agent is, for example, any agent known in the art for the alkylation of hydroxy to alkoxy or substituted alkoxy, or for the alkylation of amino to alkylamino or substituted alkylamino, for example an alkyl or substituted alkyl halide, for example a (C₁-C₄)alkyl chloride, bromide or iodide or a substituted (C₁-C₄)alkyl chloride, bromide or iodide, in the presence of a suitable base in a reaction-inert solvent and at a temperature in the range, for example, 10 to 140°C, conveniently at or near ambient temperature.

For the production of those compounds of Formula I wherein R³ is an amino-, oxy- or cyano-substituted (C₁-C₄)alkyl substituent, the reaction, preferably in the presence of a suitable base, of a Formula I compound wherein R³ is a (C₁-C₄)alkyl substituent bearing a displaceable group with an appropriate amine, alcohol or cyanide is appropriate.

The reaction is preferably carried out in a reaction-inert solvent or diluent and at a temperature in the range, for example, 10 to 100°C, conveniently at or near ambient temperature.

For the production of those compounds of Formula I wherein R³, R⁵, or R⁶ is a carboxy substituent or a substituent which includes a carboxy group, the hydrolysis of a Formula I compound wherein R³, R⁵, R⁶ is a (C₁-C₄)alkoxycarbonyl substituent or a substituent which includes a (C₁-C₄)alkoxycarbonyl group is desirable.

The hydrolysis may conveniently be preformed, for example, under basic conditions such as an alkali metal hydroxide mediated hydrolysis as illustrated In the accompanying Examples.

For the production of those compounds of Formula I wherein R³ is amino, (C₁-C₄)alkylamino, di-[(C₁-C₄)alkyl]amino, pyrrolidin-1-yl, piperidino, morpholino, piperazin-1-yl, 4-(C₁-C₄)alkylpiperazin-1-yl or (C₁-C₄)alkythio, the reaction, conveniently in the presence of a suitable base, of a Formula I compound wherein R³ is a displaceable group with an appropriate amine or thiol is preferred.

The reaction is preferably carried out in a reaction-inert solvent or diluent and at a temperature in the range, for example, 10 to 180°C, conveniently in the range 100 to 150°C.

For the production of those compounds of Formula I wherein R³ is 2-oxopyrrolidin-1-yl or 2-oxopiperidin-1-yl, the cyclisation, in the presence of a suitable base, of a Formula I compound wherein R³ is a halo-(C₂-C₄)alkanoylamino group is convenient.

The reaction is preferably carried out in a reaction-inert solvent or diluent and at a temperature in the range, for example, 10 to 100°C, conveniently at or near ambient temperature.

For the production of compounds of Formula I in which R³ is carbamoyl, substituted carbamoyl, alkanoyloxy or substituted alkanoyloxy, the carbamoylation or acylation of a Formula I compound wherein R³ is hydroxy is convenient.

Suitable acylating agents are for example any agent known in the art for acylation of hydroxyaryl moieties to alkanoyloxy aryl. For example, (C₂-C₄)alkanoyl halides, (C₂-C₄)alkanoyl anhydrides or mixed anhydrides, and suitable substituted derivatives thereof may be employed typically in the presence of a suitable base. Altematively, (C₂-C₄)alkanoic acids or suitably substituted derivatives thereof may be coupled with a Formula I compound wherein R³ is hydroxy With the aid of a condensing agent such as a carbodiimide. For the production of those compounds of Formula I in which R³ is carbamoyl or substituted carbamoyl, suitable carbamoylating agents are for example a cyanate or an alkyl or arylisocyanate, typically in the presence of a suitable base. Alternatively a suitable intermediate such as the chloroformates or imidazolylacarbonyl derivative of a heteroaryl-fused pyrimidine of Formula I In which R³ is hydroxy may be generated, for example by treatment of said derivative with phosgene (or a phosgene equivalent) or carbonyldiimidazole. The resulting intermediate may then be reacted with an appropriate amine or substituted amine to produce the desired carbamoyl derivatives.

For the production of heteroaryl-fused pyrimidine derivatives of Formula I wherein R³ is aminocarbonyl or a substituted aminocarbonyl, the aminolysis of a suitable intermediate derived from a heteroaryl-fused pyrimidine of Formula I in which R³ is carboxy is preferred.

The activation and coupling of a Formula I compound wherein R³ is carboxy may be performed by a variety of methods known to those skilled In the art. Suitable methods include activation of the carboxyl as an acid halide, azide, symmetric or mixed anhydride, or active ester of appropriate reactivity for coupling with the desired amine. Examples of such types of intermediates and their production and use in couplings with amines may be found extensively in the literature; for example M. Bodansky and A. Bodansky, "The Practice of Peptide Synthesis", Springer,-Verlag, New York, 1984.

The resulting Formula I compounds may be isolated and purified by standard methods, such as solvent removal and recrystallization or chromatography, if desired.

Optionally substituted indole and indolines, useful in the practice of the invention, and methods for their preparation, are described in co-pending United States application serial number 08/200,359, incorporated herein by reference. In addition to methods described therein the preparation of various indolines, indoles, oxindoles, and isatins useful as intermediates are further described in "Heterocyclic Compounds with indole and Carbazole Systems", W. C. Sumpter and F. M. Miller, in Vol. 8 of "The Chemistry of Heterocyclic Compounds" Series, Interscience Publishers Inc., N.Y., 1954 and references contained therein.

Substituted anilines, useful in the practice of the invention, and methods for their preparation, are described in co-pending United States application 08/413,300 (WO 96/30347) and PCT application no. PCT/IB95/00436 (WO96/30347), incorporated herein by reference.

Compounds of the formula ZH wherein ZH is QNH₂, useful in the practice of the invention, and methods for their preparation, are described in European Patent application serial no. EP 0 496 617 A1 incorporated herein by reference.

Certain compounds of Formula I can exist in solvated as well as unsolvated forms such as, for example, hydrated forms. It Is to be understood that the Invention encompasses all such solvated as well as unsolvated forms which possess activity against hyperproliferative diseases.

A suitable pharmaceutically-acceptable salt of a heteroaryl-fused pyrimidine derivative of the invention is, for example, an acid-addition salt of a heteroaryl-fused pyrimidine derivative of the invention which is sufficiently basic, for example an acid-addition salt with, for example, an inorganic or organic acid, for example, hydrochloric, hydrobromic, sulphuric, phosphoric, methanesulfonic, benzenesulfonic, trifluoroacetic, citric, lactic or maleic acid. In addition a suitable pharmaceutically-acceptable base-addition salt of a heteroaryl-fused pyrimidine derivative of the invention which is sufficiently acidic is an alkali metal salt, for example a lithium, sodium or potassium salt; an alkaline earth metal salt, for example a calcium or magnesium salt; an ammonium salt; or a salt with an organic base which affords a physiologically-acceptable cation for example a salt with methylamine, dimethylamine, trimethylamine, piperidine, morpholine or tris-(2-hydroxyethyl)amine. All such salts are within the scope of this invention and they can be prepared by conventional methods. For example, they can be prepared simply by contacting the acidic and basic entities, usually in a stoichiometric ratio, in either an aqueous, non-aqueous or partially aqueous medium, as appropriate. The salts are recovered either by filtration, by precipitation with a non-solvent, preferably an ethereal or hydrocarbon solvent, followed by filtration, by evaporation of the solvent, or, in the case of aqueous solutions, by lyophilization, as appropriate.

Some of the compounds of Formula I have asymmetric carbon atoms. Such diasteromeric mixtures can be separated into their individual diastereomers on the basis of their physical chemical differences by methods known per se., for example, by chromatography and/or fractional crystallization. Enantiomers can be separated by converting the enantiomeric mixtures into a diastereomeric mixture by reaction with an appropriate optically active compound (e.g., alcohol), separating the diastereomers and converting (e.g., hydrolyzing) the individual diastereomers to the corresponding pure enantiomer. All such isomers, including diastereomers and enantiomers are considered as part of the invention.

The compounds of this invention are potent inhibitors of the erbB family of oncogenic and protooncogenic protein tyrosine kinases such as epidermal growth factor receptor (EGFR), erbB2, HER3, or HER4 and thus are all adapted to therapeutic use as antiproliferative agents (e.g., anticancer) in mammals, particularly humans. In particular, the compounds of this invention are therapeutants or prophylactics for the treatment of a variety of human tumors (renal, liver, kidney, bladder, breast, gastric, ovarian, colorectal, prostate, pancreatic, lung, vulval, thyroid, hepatic carcinomas, sarcomas, glioblastomas, various head and neck tumors), and other hyperplastic conditions such as benign hyperplasia of the skin (e.g., psoriasis) or prostate (e.g., BPH). It is in addition expected that a heteroaryl-fused pyrimidine of the present invention may possess activity against a range of leukemias and lymphoid malignancies.

The compounds of Formula I may also be expected to be useful in the treatment of additional disorders in which aberrant expression ligand/receptor interactions, activation or signalling events related to various protein tyrosine kinases, (e.g., IGF-receptors) whose activity is inhibited by the agents of Formula I, are involved.

Such disorders may include those of neuronal, glial, astrocytal, hypothalamic, and other glandular, macrophagal, epithelial, stromal, and blastocoelic nature In which aberrant function, expression, activation or signalling of the tyrosine kinases may be involved. In addition, compounds of Formula I may have therapeutic utility in inflammatory, angiogenic and immunologic disorders involving both identified and as yet unidentified tyrosine kinases which are inhibited by compounds of Formula I.

The in vitro activity of these compounds in inhibiting the receptor tyrosine kinase (and thus subsequent proliferative response, e.g., cancer) may be determined by a procedure as detailed below. Activity of compunds of Formula I in vitro can be determined by the amount of Inhibition of the phosphorylation of an exogenous substrate (e.g., Lys₃ - Gastrin or polyGluTyr (4:1) random copolymer (I. Posner et. al., J. Biol. Chem. 267 (29), 20638-47 (1992)) on tyrosine by epidermal growth factor receptor kinase by a test compound relative to a control. Affinity purified, soluble human EGF receptor (96 ng) is obtained according to the procedure In G. N. Gill, W. Weber, Methods in Enzymology 146, 82-88 (1987) from A431 cells (American Type Culture Collection, Rockville, MD) and preincubated in a microfuge tube with EGF (2µg/ml) in phosphorylation buffer + vanadate (PBV: 50 mM HEPES, pH 7.4; 125 mM NaCl; 24 mM MgCl₂; 100µM sodium orthovanadate), in a total volume of 10µl, for 20-30 minutes at room temperature. The test compound, dissolved in dimethylsulfoxide (DMSO), is diluted in PBV*, and 10µl is mixed with the EGF receptor /EGF mix, and incubated for 10-30 minutes at 30°C. The phosphorylation reaction is initiated by addition of 20µl ³³P-ATP/ substrate mix (120µM Lys₃-Gastrin (sequence in single letter code for amino acids, KKKGPWLEEEEEAYGWLDF), 50 mM Hepes pH 7.4, 40µM ATP, 2 µCi γ-[³³P]-ATP) to the EGFr/EGF mix and Incubated for 20 minutes at room temperature. The reaction is stopped by addition of 10µl stop solution (0.5 M EDTA, pH 8; 2mM ATP) and 6 µl 2N HCI. The tubes are centrifuged at 14,000 RPM, 4°C, for 10 minutes. 35 µl of supernatant from each tube is pipetted onto a 2.5 cm circle of Whatman P81 paper, bulk washed four times in 5% acetic acid, 1 liter per wash, and then air dried. This results in the binding of substrate to the paper with loss of free ATP on washing. The [³³P] incorporated is measured by liquid scintillation counting. Incorporation in the absence of substrate (e.g., lys₃-gastrin) is subtracted from all values as a background and percent inhibition is calculated relative to controls without test compound present.

Such assays carried out with a range of doses of test compounds allow the determination of an approximate IC₅₀ value for the in vitro inhibition of EGFR kinase activity. Although the inhibitory properties of the compounds of Formula I vary with structural change as expected, In general, the activity exhibited by these agents determined in the manner described above is in the range of IC₅₀=0.0001-30 µM.

Activity of compounds of Formula I in vivo can be determined by the amount of inhibition of tumor growth by a test compound relative to a control. The tumor growth inhibitory effects of various compounds are measured according to the methods of Corbett T. H., et al. "Tumor Induction Relationships in Development of Transplantable Cancers of the Colon in Mice for Chemotherapy Assays, with a Note on Carcinogen Structure", Cancer Res., 35, 2434-2439 (1975) and Corbett, T. H., et al., "A Mouse Colon-tumor Model for Experimental Therapy", Cancer Chemother. Rep. (Part 2)", 6, 169-186 (1975), with slight modifications. Tumors are induced in the left flank by s.c. injection of 1 X 10⁶ log phase cultured tumor cells (human MDA-MB-468 breast or human HN5 head and neck carcinoma cells) suspended in 0.10 ml RPMI 1640. After sufficient time has elapsed for the tumors to become palpable (2-3 mm in diameter) the lest animals (athymic mice) are treated with compound (formulated by dissolution in DMSO typically at a concentration of 50 to 100 mg/mL followed by 1:9 dilution into 0.1% Pluronic® P105 in 0.9% saline) by the intraperitoneal (ip) or oral (po) routes of administration twice daily (i.e., every 12 hours) for 5 to 20 consecutive days. In order to determine an anti-tumor effect, the tumor is measured in millimeters with Vemier calipers across two diameters and the tumor size (mg) is calculated using the formula: Tumor weight = (length x [width]²)/2, according to the methods of Geran, R.l., et al. "Protocols for Screening Chemical Agents and Natural Products Against Animal Tumors and Other Biological Systems", Third Edition, Cancer Chemother. Results are expressed as percent inhibition, according to the formula: Inhibition (%) = (TuW_{control} - TuWₜₑₛₜ)/TuW_{control} x 100%. The flank site of tumor implantation provides reproducible dose/response effects for a variety of chemotherapeutic agents, and the method of measurement (tumor diameter) is a reliable method for assessing tumor growth rates.

Administration of the compounds of this invention can be via any method which enables delivery of the compounds to the site of action (e.g., cancer cells). These methods include oral routes, intraduodenal routes, parenteral injection (including intravenous, subcutaneous, intramuscular, intravascular or infusion), topical administration, etc.

The amount of heteroaryl-fused pyrimidine derivative administered will, of course, be dependent on the subject being treated, on the severity of the affliction, on the manner of administration and on the judgement of the prescribing physician. However an effective dosage is in the range of approximately 0.1-100 mg/kg, preferably 1 to 35 mg/kg in single or divided doses. For an average 70kg human, this would amount to 0.05 to 7 g/day, preferably 0.2 to 2.5 g/day.

The composition may, for example, be in a form suitable for oral administration as a tablet, capsule, pill, powder, sustained release formulations, solution, suspension, for parenteral injection as a sterile solution, suspension or emulsion, for topical administration as an ointment or cream or for rectal administration as a suppository. The pharmaceutical composition may be in unit dosage forms suitable for single administration of precise dosages. The pharmaceutical compositions will include a conventional pharmaceutical carrier or excipient and a compound according to the invention as an active ingredient. In addition, it may include other medicinal or pharmaceutical agents, carriers, adjuvants, etc.

Pharmaceutical compositions according to the invention may contain 0.1%-95% of the compound, preferably 1%-70%. In any event, the composition or formulation to be administered will contain a quantity of a compound according to the invention in an amount effective to alleviate or reduce the signs in the subject being treated, i.e., proliferative diseases, over the course of the treatment.

Exemplary parenteral administration forms include solutions or suspensions of a compound according to the invention Formula I in sterile aqueous solutions, for example aqueous propylene glycol or dextrose solutions are employed. Such dosage forms can be suitably buffered, if desired.

Suitable pharmaceutical carriers include inert diluents or fillers, water and various organic solvents. These pharmaceutical compositions can, if desired, contain additional ingredients such as flavorings, binders, excipients and the like. Thus for oral administration, tablets containing various excipients, such as citric acid can be employed, together with various disintegrants such as starch, alginic acid and certain complex silicates and with binding agents such as sucrose, gelatin and acacia. Additionally, lubricating agents such as magnesium stearate, sodium lauryl sulfate and talc are often useful for tableting purposes. Solid compositions of a similar type may also be employed in soft and hard filled gelatin capsules. Preferred materials therefore include lactose or milk sugar and high molecular weight polyethylene glycols. When the aqueous suspensions or elixirs are desired for oral administration the essential active ingredient therein may be combined with various sweetening or flavoring agents, coloring matters or dyes and, if desired, emulsifying agents or suspending agents, together with diluents such as water, ethanol, propylene glycol, glycerin, or combinations thereof.

Methods of preparing various pharmaceutical compositions with a certain amount of active ingredient are known, or will be apparent, to those skilled in this art. For examples, see Remington's Pharmaceutical Sciences., Mack Publishing Company, Easter, Pa., 15th Edition (1975).

The anticancer treatment described above may be applied as a sole therapy or may involve, in addition to the heteroaryl-fused pyrimidine derivative of the invention. one or more other antitumor substances. Such conjoint treatment may be achieved by way of the simultaneous, sequential, cyclic or separate dosing of the individual components of the treatment.

It should be understood that the invention is not limited to the particular embodiments shown and described herein, but that various changes and modifications may be made without departing from the spirit and scope of this novel concept as defined by the following claims.

### Example 1

### (3-Ethynyl-phenyl)-(7H-pyrrolo[2,3-d]pyrimidin-4-yl)-amine hydrochloride:

To 4-chloro-7H-pyrrolo[2,3-d]pyrimidine (10.0 g, 0.065 mol) in dry pyridine (90 ml) was added m-aminophenylacetylene (9.2 g, 0.078 mol), and the mixture was heated in an 85°C oil bath for 2 days. The reaction mixture was cooled to ambient temperature and concentrated in vacuo. The resulting residue was purified by flash chromatography on silica gel (375 g, 40µm mesh) using 5% methanol/CH₂Cl₂ to afford the title compound as a pink-orange solid (1.9 g, 12% ). HRMS: Calcd. 235.0984, Found 235.1000; anal. RP18-HPLC RT: 3.48 min.

The above compound was dissolved in a minimal amount of methanol and a solution of HCl in ethyl ether (HCl bubbled into 2 ml ethyl ether) was added dropwise until the mixture remained cloudy. The precipitated HCl salt was dried in vacuo, washed once with ethyl ether, and dried in vacuo to constant mass. MP: 196-198°C.

### Example 2

### (3-Chloro-phenyl)-(7H-pyrrolo[2,3-d]pyrimidin-4-yl)-amine

Following the procedure described in Example 1, the title compound was prepared from 4-chloro-7H-pyrrolo[2,3-d]pyrimidine and 3-chloroaniline(3.4%). LC-MS: 245 (MH⁺); anal. RP18-HPLC RT: 3.74 min; HCI salt MP: 227-228°C.

### Example 3

### 4-(6-Chloro-2,3-dihydro-indol-1-yl)-7H-pyrrolo[2,3-d]pyrimidine hydrochloride:

Following the procedure described in Example 1, the title compound was prepared from 4-chloro-7H-pyrrolo[2,3-d]pyrimidine and 6-chloro-2,3-dihydroindol-1-yl (4.3%). HRMS: Calcd. 271.0750, Found 271.0729; anal. RP18-HPLC RT: 4.88 min; HCl salt MP: 266°C (dec).

### Example 4

### (7H-Pyrrolo[2,3-d]pyrimidin-4-yl)(-m-tolyl-)amine hydrochloride:

Following the procedure described in Example 1, the title compound was prepared from 4-chloro-7H-pyrrolo[2,3-d]pyrimidine and m-toluidine (34%). HRMS: Calcd. 225.1140, Found 225.1131; anal. RP18-HPLC RT: 3.45 min; HCl salt MP: 219°C.

### Example 5

### (1H-Indol-5-yl)-(7H-pyrrolo[2,3-d]pyrimidin-4-yl)-amine hydrochloride:

Following the procedure described In Example 1, the title compound was prepared from 4-chloro-7H-pyrrolo[2,3-d]pyrimidine and 5-aminoindole (7%). HRMS: Calcd. 250.1093, Found 250.1081; anal. RP18-HPLC RT: 2.58 min; HCl salt MP: 218-221°C.

### Example 6

### Phenyl-(7H-pyrrolo[2,3-d]pyrimidin-4-yl)-amine

Utilizing a procedure analogous to that described in Example 1, this product was prepared in 16% yield from 4-Chloro-7H-pyrrolo[2,3-d]pyrimidine (1.0 eq) and aniline (5.0 eq) in pyridine. (M.P. 234-236°C; GC-MS: 211(MH⁺); anal. RP18-HPLC RT: 3.11 min.)

The compounds of Examples 7-10 were made according to the method of claim 6 from the appropriate starting materials.

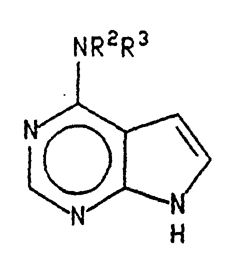

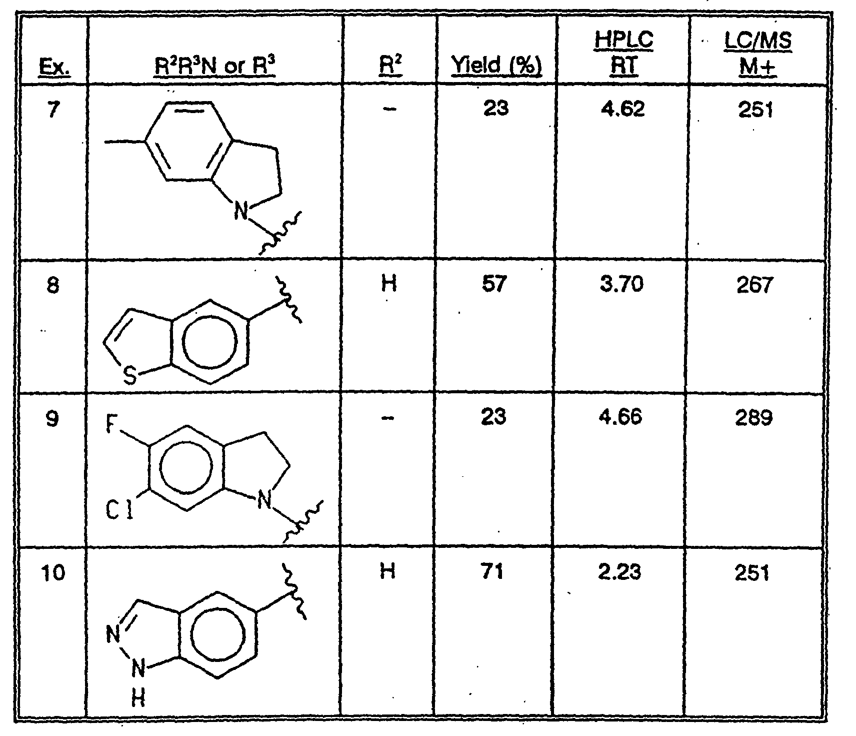

### Example 11

### 1-(4-[m-Tolylamino]-1-(pyrrolo[2,3-d]pyrimidin-7-yl)-ethanone hydrochloride:

To (3-methyl-phenyl)-(7H-pyrrolo[2,3-d]pyrimidin-4-yl)-amine (Example 4) (0.168 g, 0.75 mmol), dissolved in hot acetonitrile (7 ml), was added sodium hydride (36 mg, 0.90 mmol, 60% dispersion in mineral oil). After stirring at ambient temperature for 0.75 hours, acetyl chloride (0.11 ml, 1.5 mmol) was added and stirring was continued for 48 hours. The mixture was concentrated in vacuo, triturated in hot ethyl acetate and filtered. The filtrate was concentrated in vacuo to give an orange solid residue. The solid was triturated in CH₂Cl₂ and filtered to afford the title compound as a light yellow solid (0.11 g, 55%). LC-MS: 267 (MH⁺); anal. RP18-HPLC RT: 3.53 min.

### Example 12

### (5-Iodo-7H-pyrrolo[2,3-d]pyrimidin-4-yl)-(m-tolyl)-amine

To 1-(4-tolylamino-pyrrolo[2,3-d]pyrimidin-7-yl)-ethanone (113 mg, 0.42 mmol), in dry methanol (4 ml), and CH₂Cl₂ (1 ml), was added Na₂CO₃ (45 mg, 0.42 mmol). After stirring at ambient temperature for 0.75 hours, N-iodosuccinimide (190 mg, 0.85 mmol) was added and stirring was continued for 48 hours. The mixture was concentrated in vacuo and partitioned between CH₂Cl₂ and H₂O. The organic phase was washed twice with H₂O, dried over Na₂SO₄ and concentrated in vacuo. The resulting residue was purified by flash chromatography on silica gel (7 g, 40 µm mesh) using 2% methanol/CH₂Cl₂ to afford the title compound as olive needles (6mg, 4%). LC-MS: 351 (MH⁺); anal. RP18-HPLC RT: min.

### Example 13

### (7-Benzenesulfonyl-7H-pyrrolo[2,3-d]pyrimidin-4-yl)-(3-ethynyl-phenyl)-amine

To 4-chloro-7H-pyrrolo[2,3-d]pyrimidine (1.0 g, 0.0065 mol) in dry THF (10 ml) under nitrogen at -78°C was added dropwise via syringe over 15 minutes n-butyllithium (2.5 M in hexane; 2.88 ml, 0.0072 mol). The cooling bath was removed and the solution was stirred for 1 hour. The resulting pyrrolo anion salt precipitated as a very fine white solid in a cloudy colorless solution. After the suspension was recooled to 78°C, benzenesulfonyl chloride (1.26 g, 0.0072 mol) was added neat via syringe. The resulting yellow reaction mixture was allowed to warm slowly to room temperature overnight. The grey-white suspension was poured into 2% aqueous sodium bicarbonate (50 mL) and extracted with two times with diethyl ether (20mL). The combined extracts were washed with water and dried (potassium carbonate) and evaporated to give a light amber oil which crystallized from ether, the product was collected by filtration to 1.4 g (74%) of white solid. LC-MS= 294 (MH+) RP18-HPLC RT: 4.40 min.

The above compound was dissolved in methanol and m-aminophenyl acetylene (0.159 g, 0.0013 mol), and the reaction mixture heated in an 85°C oil bath for 2 days. The reaction mixture was cooled to ambient temperature and concentrated in vacuo. The residue was titurated with diethyl ether to produce the title product as a white solid (0.234 g, 92%). LC-MS= 375 (MH+), RP18-HPLC RT: 3.48 min.

### Example 14

### 4-(6-Chloro-2,3-dihydro-indol-1-yl)-5H-pyrrolo[3,2-d]pyrimidin-6-ol

To a solution of 4-(6-Chloro-2,3-dihydro-indol-1-yl)-5-amino-6-methylacetylpyrimidine (541 mg, 1.55 mmol) in 40 mL of ethanol was added 25 mol% of 10% palladium on carbon (125 mg) and 0.11 mL of 1N HCl (1.55 mmol). The reaction mixture was hydrogenated for 3 hours at 50 psi (3.4 x 10⁵ Pa). The reaction mixture was filtered through Celite and concetrated in vacuo. The brown residue was slurried in methanol and the white solid title product was filtered off (279 mg, 63%)LC-MS= 287 (M+), RP18-HPLC RT: 5.61 min. MP: 250°C(dec).

### Example 15

### (3-Ethynyl-phenyl)-[7-(2-morpholin-4-yl-ethyl)-7H-pyrrolo[2,3-d]pyrimidin-4-yl]-amine

To a solution of 184 mg (1.4 mmol) of 4-(2-hydroxyethyl)morpholine in 10 mL of toluene was added 276 mg (2.0 mmol) of anhydrous potasium carbonate and then 32 mg (1.3 mmol) of 97% sodium hydride. After 30 minutes 343 mg (1.0 mmol) of sulfonylated 4-chloro-7H-pyrrolo[2,3-d]pyrimidine was added and the reaction mixture was heated at 100°C for 2 hours. The reaction mixture was then partitioned between ethyl acetate and water and the aqueous layer was extracted with two additional portions of ethyl acetate. The combined organic phases were washed with water, dried over magnesium sulfate and concentrated in vacuo. The residue was chromatographed on silica gel using 10% methanol/methylene chloride to give an amber oil (140 mg, 55%). LC-MS 267 (M+)

The above product was dissolved in methanol and m-aminophenyl acetylene (0.123 g, 0.001 mol), and the reaction mixture was heated in a sealed tube in a 120°C oil bath for 12 hours. The reaction mixture was cooled to amblent temperature and concentrated in vacuo. The residue was titurated with diethyl ether to produce the title product as a white solid (0.135 g, 74%). LC-MS= 348 (MH+), RP18-HPLC RT: 3.33 min.

### Example 16

### (3-Ethynyl-phenyl)-[7-(2-methoxy-ethyl)-7H-pyrrolo[2,3-d]pyrimidin-4-yl]-amine

Utilizing a procedure analogous to that described in Example 14, this product was prepared in 81 % yield from 4-chloro-7-(2-methoxy-ethyl)-7H-pyrrolo[2,3-d]pyrimidine (1.0 eq) and m-aminophenyl acetylene (1.2 eq) in methanol. M.P. 240-241 °C; LC-MS: 292(MH+); RP18-HPLC RT: 4.16 min.

### Example 17

### (3-Ethynyl-phenyl)-{7-[2-(2-methoxy-ethoxy)-ethyl]-7H-pyrrolo[2,3-d]pyrimidin-4-yl}-amine

Utilizing a procedure analogous to that described in Example 14, this product was prepared in 81% yield from 4-chloro-7-[2-(2-methoxy-ethoxy)-ethyl]-7H-pyrrolo[2,3-d]pyrimidine (1.0 eq) and m-aminophenyl acetylene (1.2 eq) in methanol. M.P. 240-241 °C; LC-MS: 336 (M+); RP18-HPLC RT: 4.29 min.

### Example 18

### (7-Allyl-pyrrolo[2,3-d]pyrimidin-4-yl)-(3-ethynyl-phenyl)-amine hydrochloride

To 4-chloro-7H-pyrrolo[2,3-d]pyrimidine (1.3 g, 8.5 mmol) in dry THF (30 ml) was added sodium hydride (1.0 g, 0.25 mmol, 60% dispersion in mineral oil). After stirring at ambient temperature for 1 hour, allyl iodide (0.93 ml, 10 mmol) was added and stirring was continued for 48 hours. The reaction mixture was concentrated in vacuo, triturated in hot ethyl acetate, and filtered. The filtrate was concentrated in vacuo to give an orange solid residue. The solid was triturated in methylene chloride and filtered to afford 4-chloro-7-allyl-pyrrolopyrimidine as a light yellow powder (0.58 g, 36%). TS-MS: 194 (MM+); anal. RP18-HPLC RT: min. To 4-chloro-7-allyl-pyrrolopyrimidine (0.5 g, 2.6 mmol) in dry methanol (5 ml) was added m-aminophenyl acetylene (0.36 g, 3.1 mmol). The suspension was heated in a sealed pressure tube at 125°C for 20 hours. The reaction mixture was cooled to ambient temperature and concentrated in vacuo. The resulting oil was purified by flash chromatography on silica gel (50 g, 40 mm mesh) using 3% methanol/methylene chloride to afford the title product as a yellow powder (0.29 g, 41%). TS-MS: 275 (MH+); anal. RP18-HPLC RT: min.

### Example 19

### (3-Ethynyl-phenyl)-(7-methyl-pyrrolo[2,3-d]pyrimidin-4-yl)-amine hydrochloride

Following the procedure described in Example 1, the title compound was prepared from 4-chloro-7H-pyrrolo[2,3-d]pyrimidine and methyl iodide, and m-aminophenyl acetylene (75%). TS-MS: 249 (MH+); anal. RP18-HPLC RT: min.

### Example 20

### (5-Bromo-7H-pyrrolo[2,3-d]pyrimidin-4-yl)-(3-ethynyl-phenyl)-amine

To 4-chloro-7H-pyrrolo[2,3-d]pyrimidine (0.21 g, 1.4 mmol) in dry methylene chloride (10 ml) was added N-bromosuccinimide (0.26 g, 1.5 mmol) at ambient temperature. The reaction mixture was stirred for 18 hours, and the resulting solid filtered with methylene chloride washes and dried in vacuo to afford 5-bromo-4-chloro-7H-pyrrolo[2,3-d]pyrimidine as a tan powder (0.28 g, 88%). GC-MS: 233 (MH+), RT: 4.42 min.

To 5-bromo-4-chloro-7H-pyrrolo[2,3-d]pyrimidine (0.13 g, 0.57 mmol) in dry methanol (2 ml) was added m-aminophenyl acetylene (0.08 g, 0.68 mmol). The suspension was heated in a sealed pressure tube at 125°C for 18 hours. The reaction mixture was cooled to ambient temperature and concentrated in vacuo. The resulting oil was purified by flash chromatography on silica gel (10 g, 40 mm mesh) using 3% methanol/methylene chloride to afford the title compound as a yellow powder (71 mg, 39% ). TS-MS: 314 (MH+); anal. RP18-HPLC RT: min.

### Example 21

### (3-Ethynyl-phenyl)-(5-iodo-7H-pyrrolo[2,3-d]pyrimidin-4-yl)-amine

To(3-methyl-phenyl)-(7H-pyrrolo[2,3-d]pyrimidin-4-yl)amine(17 mg, 0.076 mmol) in dry methylene chloride (1ml) was added N-iodosuccinimide (19 mg, 0.083 mmol). The reaction mixture was stirred at ambient temperature for 2 hours then filtered with a methylene chloride wash and dried in vacuo to afford the title compound as a grey-tan powder (12 mg, 46% ). TS-MS: 351 (MH+); anal. RP18-HPLC RT: min.

### Example 22

### 4-(3-Ethynyl-phenylamino)-7H-pyrrolo[2,3-d]pyrimidine-5-carboxylic acid

To 5-bromo-4-chloro-7H-pyrrolo[2,3-d]pyrimidine (0.87 g, 3.7 mmol) in dry THF (29 ml) cooled in a dry ice-acetone bath was added dropwise n-butyllithium (3.4 ml, 8.4 mmol, 2.5 M in hexanes). The reaction mixture was stirred for 1 hour then quenched by bubbling In CO₂. To the resulting olive suspension was added water (1 ml) and stirred for 5 minutes at ambient temperature. The reaction mixture was concentrated in vacuo, triturated with ethyl acetate, and dried in vacuo to afford 4-chloro-7H-pyrrolo[2,3-d]pyrimidine-5-carboxylic acid as an avocado powder (0.80 g, 74% ). TS-MS: 198 (MH+); anal. RP18-HPLC RT: min.

To 4-chloro-7H-pyrrolo[2,3-d]pyrimidine-5-carboxylic acid (0.38 g, 1.9 mmol) in dry methanol (4 ml) was added m-aminophenyl acetylene (0.47 g, 4.0 mmol). The suspension was heated in a sealed pressure tube at 125°C for 18 hours. The reaction mixture was cooled to ambient temperature, filtered with methylene chloride washes and dried in vacuo to afford the title compound as a tan powder (0.30 g, 54% ). TS-MS: 278 (MH+); anal. RP18-HPLC RT: min.

### Example 23

### (3-Ethynyl-phenyl)-(5-methyl-7H-pyrrolo[2,3-d]pyrimidin-4-yl)-amine hydrochloride

To 5-bromo-4-chloro-7H-pyrrolopyrimidine (0.28 g, 1.2 mmol) in dry THF (9 ml) cooled in a dry ice-acetone bath was added dropwise n-butyllithium (1.1 ml, 2.7 mmol, 2.5 M in hexanes). The reaction mixture was stirred for 1 hour, then methyl iodide (0.12 ml, 1.9 mmol) was added. The solution was stirred for 1 hour at ambient temperature, and water (1 ml) was added. The reaction mixture was concentrated in vacuo, and diluted with ethyl acetate and water. The organic phase was washed twice with water, dried over sodium sulfate and concentrated in vacuo to afford 4-chloro-5-methyl-7H-pyrrolo[2,3-d]pyrimidine (0.17 g, 85%). GC-MS: 167(M+), RT: 3.15 min.

To 4-chloro-5-methyl-7H-pyrrolopyrimidine (0.17 g, 1,0 mmol) in dry methanol (3 ml) was added m-aminophenyl acetylene (0.14 g, 1.2 mmol). The suspension was heated in a sealed pressure tube at 125°C for 18 hours. The reaction mixture was cooled to ambient temperature, and concentrated in vacuo. The resulting residue was purified by flash chromatography on silica gel (15 g, 40 mm mesh) using 5% methanol/methylene chloride to afford the title compound as a yellow solid (0.11 g, 43%). TS-MS: 249 (MH+); anal. RP18-HPLC RT: min.

### Example 24

### N-(5-Iodo-7H-pyrrolo[2,3-d]pyrimidin-4-yl)-N-m-tolyl-acetamide

To (3-methyl-phenyl)-(7H-pyrrolo[2,3-d]pyrimidin-4-yl)-amine (0.75 g, 3.4 mmol) dissolved in hot acetonitrile (30ml) was added sodium hydride (0.16 g, 4.0 mmol, 60% dispersion in mineral oil). After stirring at ambient temperature for 0.75 hours, acetyl chloride (0.48 ml, 6.7 mmol) was added.and stirring continued for 48 hours. The reaction mixture was concentrated in vacuo, triturated in hot ethyl acetate, and filtered. The filtrate was concentrated In vacuo to give an orange solid residue. The solid was purified by flash chromatography on silica gel (13 g, 40 mm mesh) using 1:3 ethyl acetate/hexanes to afford 1-(4-m-tolylamino-pyrrolo[2,3-d]pyrimidin-7-yl)-ethanone as a yellow solid (0.21 g). TS-MS: 309 (MH+); anal. RP18-HPLC RT: min.

To 1-(4-m-tolylamino-pyrrolo[2,3-d]pyrimidin-7-yl)-ethanone (0.21 g, 0.79 mmol) in dry methylene chloride (5 ml) and dry methanol (2 ml) was added sodium carbonate (0.17 g, 1.6 mmol). After stirring at ambient temperature for 0.75 hours, N-lodosuccinimde (0.35 g, 1.6 mmol) was added. The reaction mixture was stirred at ambient temperature for 48 hours then concentrated in vacuo. The residue was diluted with methylene chloride and water. The water phase was extracted once with methylene chloride The organic phase was washed twice with water, dried over sodium sulfate , and concentrated in vacuo. The resulting residue was purified by flash chromatography on silica gel (11 g, 40 mm mesh) using 2% methanol/methylene chloride to afford the title compound as a yellow solid (30 mg ). TS-MS: 393 (MH+); anal. RP18-HPLC RT: min.

### Example 25

### 4-(3-Ethynyl-phenylamino)-7H-pyrrolo[2,3-d]pyrimidine-5-carboxylic acid methyl ester hydrochloride

To 4-(3-ethynyl-phenylamino)-7H-pyrrolo[2,3-d]pyrimidine-5-carboxylic acid (0.108 g, 0.39 mmol) in dry methylene chloride (2 ml) was added a solution of oxalyl chloride (0.17 ml, 1.9 mmol) in dry methylene chloride (4 ml) followed by 1 drop of dry DMF. The suspension was stirred at ambient temperature for 1 hour, then concentrated in vacuo. To the resulting solid was added dry acetone (2 ml) and dry methanol (1 ml). The solution was stirred at ambient temperature for 15 hours, then concentrated in vacuo. The residue was diluted with ethyl acetate and water, and the solid filtered and dried in vacuo to afford the title compound as a tan powder (40 mg, 35%). TS-MS: 293 (MH+); anal. RP18-HPLC RT: min.

### Example 26

### (3-ethynyl-phenylamino)-(7H-pyrrolo[2,3-d]pyrimidin-5-yl-carbonitrile

To 5-bromo-4-chloro-7H-pyrrolo[2,3-d]pyrimidine (0.31 g, 1.3 mmol) in dry THF (4 ml), cooled in a dry ice-acetone bath, was added dropwise n-butyllithium (1.3 ml, 3.3 mmol, 2.5 M in hexanes). The reaction mixture was stirred for 1 hour, then p-toluenesulphonyl cyanide (0.44 g, 2.4 mmol) suspended in dry THF (7 ml) was added. The solution was stirred for 18 hours at ambient temperature then diluted with aqueous ammonium chloride. The phases were separated and the organic phase washed with water and aqueous NaCl. The organic phase was dried over sodium sulfate , and concentrated in vacuo. The resulting residue was purified by flash chromatography on silica gel (15 g, 40 mm mesh) using 3% methanol/methylene chloride to afford 4-chloro-5-cyano-7H-pyrrolo[2,3-d]pyrimidine as a yellow solid (52 mg).

To 4-chloro-5-cyano-7H-pyrrolo[2,3-d]pyrimidine (52 mg, 0.29 mmol) in dry methanol (3 ml) was added m-aminophenyl acetylene (41 m g, 0.35 mmol). The suspension was heated in a sealed pressure tube at 125°C for 18 hours. The reaction mixture was cooled to ambient temperature, filtered with a small amount of methanol, and dried in vacuo to afford the title compound as a white solid (27mg, 36%). TS-MS: 260 (MH+); anal. RP18-HPLC RT: 3.70 min.

### Example 27

### (3-Ethynyl-phenyl)-(5-methylsulfanyl)-7H-pyrrolo[2,3-d]pyrimidin-4-yl)-amine

To 5-bromo-4-chloro-7H-pyrrolo[2,3-d]pyrimidine (0.18 g, 0.77 mmol) in dry THF (2 ml), cooled in a dry ice-acetone bath, was added dropwise n-butyllithium (0.77 ml, 1.9 mmol, 2.5 M in hexanes). The mixture was stirred for 1 hour, then dimethyldisulfide (0.077 mL, 0.77 mmol) suspended in dry THF (1 ml) was added. The solution was stirred for 2.5 hours at -78°C and then diluted with aqueous NH₄Cl. The phases were separated and the water extracted 2 times with ethyl acetate. The combined organic phases were dried over Na₂SO₄, and concentrated in vacuo to afford 4-chloro-5-methylsulfanyl-7H-pyrrolo[2,3-d]pyrimidine as an orange solid (150 mg).

To 4-chloro-5-methylsulfanyl-7H-pyrrolo[2,3-d]pyrimidine (150 mg, 0.75 mmol) in dry methanol (2 ml) was added m-aminophenyl acetylene (110 mg, 0.90 mmol). The solution was heated in a sealed pressure tube at 125°C for 5.5 hours. The reaction mixture was cooled to ambient temperature, filtered with a small amount of methanol and dried in vacuo to afford the title compound as a tan powder (57 mg, 27%). TS-MS: 281 (MH⁺); anal. RP18-HPLC RT: 4.74 min.

## Claims

1. A compound of the formula 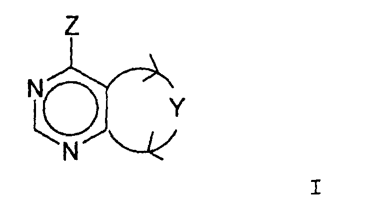 and stereoisomers, and pharmaceutically acceptable salts thereof, wherein Y, in the direction shown by the arrow in formula I, is selected from CR³=CR³-NR⁴- and -NR⁴-CR³=CR³- ;
Z is NR¹R² wherein R¹ is H and R² is phenyl substituted by (R⁵)ₘ or Q or R¹R²N is a group of the formula 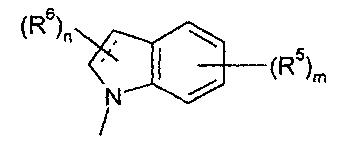 wherein the dotted line represents an optional double bond;
each R³ is attached to a carbon atom in Y and is independently selected from
a. hydrogen, trifluoromethyl, halo, nitro, hydroxy, amino, cyano, (C₁-C₄)alkyl, (C₁-C₄)alkoxy, (C₁-C₄)alkoxycarbonyl, (C₁-C₄)alkanoyloxy, (C₁-C₄)alkanoylamino, carboxy, phenoxy, benzoyloxy, carbamoyl, mono-N-or di-N-N-di-(C₁-C₄)alkylcarbamoyl, mono-N- or di-N,N-(C₁-C₄)alkylamino, mono-N- or di-N,N-(hydroxy(C₂-C₄)alkyl)amino, mono-N-or di-N,N-((C₁-C₄)alkoxy(C₂-C₄)alkyl)amino, or such groups substituted on (C₁-C₄)alkyl;
b. hydroxy(C₂-C₄)alkoxy(C₁-C₄)alkyl, (C₁-C₄)alkoxy-(C₁-C₄)alkoxy-(C₁-C₄)alkyl, hydroxy(C₂-C₄)alkylthio(C₁-C₄), (C₁-C₄)alkoxy(C₂-C₄)alkylthio(C₁-C₄)alkyl, hydroxyamino, benzoylamino, mono-N- or di-N,N-(C₁-C₄)alkylcarbamoylmethylamino, carbamoylmethylamino, (C₁-C₄)alkoxycarbonylamino, (C₁-C₄)alkanoylamino, carboxymethylamino, (C₁-C₄)alkoxycarbonylmethylamino, (C₁-C₄)alkoxyamino, (C₂-C₄)alkanoyloxyamino, (C₁-C₄)alkylsulfonylamino, ureido,(C₁-C₄)alkoxy (C₁-C₄)alkylcarbonylamino,(C₁-C₄)alkylsulfinyl,(C₁-C₄)alkylsulfonyl, (C₁-C₄)alkoxy(C₂-C₄)alkylthio, mono-, di- or trifluoromethyloxy, (C₁-C₄)alkylenedioxy, guanidino, aminocarbonyl, mono-N- or di-N,N-(C₁-C₄)alkylaminocarbonyl, carboxymethoxy, (C₁-C₄)alkoxycarbonylmethoxy, carbamoylmethoxy, mono-N or di-N,N-(C₁-C₄)alkylcarbamoylmethoxy, mono-N- or di-N,N-(hydroxy(C₂-C₄)alkyl)carboxamido, mon-N- or di-N,N-((C₁-C₄)alkoxy (C₂-C₄)alkyl)carboxamido or bis((C₁-C₄)alkanesulfonyl)amido; or
c. (C₂-C₄)alkoxy, (C₂-C₄)alkylthio, (C₂-C₄)alkanoyloxy, (C₂-C₄)alkylamino, (C₁-C₄)alkyl(C₁-C₄)alkylenedioxy, (C₂-C₄)alkanoylamino, (C₂-C₄)alkenyl, or (C₂-C₄)alkynyl; each such group substituted with amino, halo, hydroxy, (C₂-C₄)alkanoyloxy, (C₁-C₄)alkoxy, mono-N- or di-N,N-(C₁-C₄)alkylamino, mono-N or di-N,N-(hydroxy(C₂-C₄)alkyl)amino, mono-N or di-N,N-((C₁-C₄)alkoxy(C₂-C₄)alkoxy(C₂-C₄)alkyl)amino, (C₁-C₄)alkanoylamino, carboxy(C₁-₄)alkylthio(C₄)alkoxy, carboxy, (C₁-C₄)alkoxycarbonyl, carbamoyl, mono-N or di-N,N-(C₁-C₄)alkylcarbamoyl, carboxamido, mono-N- or di-N,N-(C₁-C₄)alkylcarboxamido or mono-N- or di-N,N-(hydroxy(C₂-C₄)alkyl)carboxamido; and any phenyl in an R³ substituent is optionally mono- or di- substituted with halo, nitro, trifluoromethyl, hydroxy, (C₁-C₄)alkoxy, (C₁-C₄)alkyl, amino, mono-N-alkylamino, or N,N-dialkylamino;
R⁴ is attached to the N-atom in Y and is selected from: hydrogen, (C₁-C₄)alkyl, (C₁-C₄)alkoxycarbonyl, (C₁-C₄)alkanoyl, (C₁-C₄)alkylsulfonyl, arylsulfonyl, allyl; or a (C₂-C₄)alkyl, (C₂-C₄)alkanoyl, or C₂-C₄)alkoxycarbonyl, (C₂-C₄)alkylsulfonyl, each such group substituted with amino, halo, hydroxy, (C₂-C₄)alkanoyloxy, (C₁-C₄)alkoxy, mono-N-or di-N,N-(C₁-C₄)alkylamino, mono-N or di-N,N-(hydroxy (C₂-C₄)alkylamino, mono-N or di-N,N-((C₁-C₄)alkoxy(C₂-C₄)alkyl)amino, (C₁-C₄)alkanoylamino, carboxy, (C₁-C₄)alkoxycarbonyl, carbamoyl, mono-N- or di-N,N-(C₁-C₄)alkylcarbamoyl, carboxamido, mono-N- or di-N,N-(C₁-C₄)alkylcarboxamido or mono-N- or di-N,N-(hydroxy(C₂-C₄)alkyl)carboxamido;
each R⁵ is independently selected from mono-, di- or tri-fluoromethyl, halo, nitro, hydroxy, amino, azido, isothiocyano, (C₁-C₄)alkyl, (C₁-C₄)alkoxy, (C₂-C6)alkenyl, (C₂-C₆)alkynyl, (C₁-C₄)alkylenedioxy, cyano, trifluoromethylcarbonylamino, (C₁-C₄)alkanoylamino, (C₁-C₄)alkanoyl,N-mono- or N,N-di-(C₁-C₄)alkylamino, (C₁-C₄)alkylsulfonylamino, trifluoromethylsulfonylamino, (C₁-C₄)alkylthio, (C₁-C₄)alkylsulfinyl or (C₁-C₄)alkylsulfonyl, and said (C₁-C₄)alkylenedioxy is linked at both ends to adjacent carbons on the benzene moiety;
each R⁶ is independently selected from hydroxy, amino, N-mono- or N,N-di(C₁-C₄)alkylamino, sulfo, or (C₁-C₄)alkoxy (provided that such groups are not attached to a ring carbon which is directly adjacent to the ring N-), or R⁶ for each occurrence is independently carboxy, hydroxy(C₁-C₄)alkyl, (C₁-C₄)alkoxy(C₁-C₄)alkyl, amino(C₁-C₄)alkyl, mono-N- or di-N,N-(C₁-C₄)alkylamino(C₁-C₄)alkyl, morpholino (C₁-C₄)alkyl, 4-(C₁-C₄)alkyl-piperazin-1-yl(C₁-C₄)alkyl, carboxy(C₁-C₄)alkyl, (C₁-C₄)alkoxycarbonyl, sulfo(C₁-C₄)alkyl, pyridyl(C₁-C₄)alkyl or C₁-C₄)alkyl;
m is an interger from 1 to 3;
n is 0, 1 or 2;
p is 0 or an integer from 1-3;
Q is a 9- or 10-membered bicyclic heteroaryl cyclic moiety, or a hydrogenated derivative thereof, containing one or two nitrogen heteroatoms and optionally containing a further heteroatom selected from nitrogen, oxygen and sulphur, or Q is a 9- or 10-membered bicyclic aryl moiety, or a hydrogenated derivative thereof, which heterocyclic or aryl moiety, or hydrogenated derivatives thereof, may optionally bear one or two substituents selected from halogeno, hydroxy, oxo, amino, nitro, carbamoyl, (C₁-C₄)alkyl, (C₁-C₄)alkoxy, (C₁-C₄)alkylamino, di-[(C₁-C₄)alkyl]amino, (C₂-C₄)alkanoylamino, and (C₂-C₄)alkynyl with the proviso that when Y, in the direction shown by the arrow in formula I, is -NR⁴-CR³=CR³-, R³ = CH₃ and R⁴=H, then R⁵ is not 4-CH₃, 3,5-(CH₃)₂, 2,6-(CH₃)₂, 2-C₂H₅, 4-C₂H₅, 4-n-C₄H₉, 2-Cl, 4-Cl, 3,4-Cl₂, 2-F, or 3-CF₃.

2. The compound of claim 1 wherein each R³ is independently selected from hydrogen, hydroxy, (C₁-C₄)alkoxy, hydroxy(C₂-C₄)alkoxy, amino(C₂-C₄)alkyl, amino (C₂-C₄)alkoxy, (C₁-C₄)alkoxy(C₂-C₄)alkoxy, hydroxy(C₁-C₄)alkyl(C₁-C₄)alkylenedioxy, (C₁-C₄)alkoxy(C₁-C₄)alkyl(C₁-C₄)alkylenedioxy, mono-N- or di-N,N-(C₁-C₄)alkylamino(C₂-C₄)alkoxy, 3- or 4-(C₁-C₄)alkoxy-(2-hydroxy)-(C₃-C₄)alkoxy, carboxy(C₁-C₄)alkoxy, (C₁-C₄)alkoxy(C₁-C₄)alkanoyloxy, nitro, hydroxylamino, amino, mono-N- or di-N,N-(C₁-C₄)alkylamino, (C₁-C₄)alkanoylamino, hydroxy(C₂-C₄)alkylamino, (C₁₋C₄)alkoxy(C₂-C₄)alkylamino, (C₁-C₄)alkylsulfonamido, bis(C₁-C₄)alkanesulfonamido, di-N,N-(C₁-C₄)alkylamino(C₂-C₄)alkylamino, (C₁-C₄)alkylamino(C₂-C₄)alkylamino, (C₁-C₄)alkoxy(C₁-C₄)alkylcarbonylamino, carboxy, (C₁-C₄)alkoxycarbonyl, (C₁-C₄)alkoxycarbonyl(C₁-C₄)alkoxy, amido, mono-N- or di-N,N-(C₁-C₄)alkylaminocarbonyl, mono-N- or di-N,N-(hydroxy(C₂-C₄)alkyl)aminocarbonyl, (C₁-C₄)alkyl, hydroxy(C₁-C₄)alkyl, mono-N- or di-N,N-((C₁-C₄)alkoxy(C₁-C₄)alkyl)amino(C₁-C₄)alkyl, mono-N- or di-N,N-(C₁-C₄)alkylamino(C₁-C₄)alkyl, C₁-C₄)alkanoylamino(C₁-C₄)alkyl, (C₁-C₄)alkoxy(C₂-C₄)alkoxy(C₁-C₄)alkyl, (C₁-C₄)alkylthio, (C₁-C₄)alkoxy(C₂-C₄)alkylthio or hydroxy(C₂-C₄)alkylthio; and R⁴ is selected from hydrogen, a (C₂-C₄)alkyl, hydroxy(C₂-C₄)alkyl, or hydroxy(C₂-C₄)alkyl, amino(C₂-C₆)alkyl, (C₂-C₄)alkoxycarbonyl each such group substituted with amino, halo, hydroxy, (C₂-C₄)alkanoyloxy, (C₁-C₄)alkoxy, mono-N- or di-N,N-(C₁-C₄)alkylamino, mono-N or di-N,N-(hydroxy(C₂-C₄)alkyl)amino, mono-N or di-N,N-((C₁-C₄)alkoxy(C₂-C₄)alkyl)amino, sulfonylaryl(C₁-C₄)alkylamine, (C₁-C₄)alkanoylamino, carboxy, (C₁-C₄)alkoxycarbonyl, carbamoyl, mono-N- or di-N,N-(C₁-C₄)alkylcarbamoyl, carboxamido, mono-N- or di-N,N-(C₁-C₄)alkylcarboxamido or mono-N- or di-N,N-(hydroxy(C₂-C₄)alkyl)carboxamido.

3. The compound of claim 2 wherein Y, in the direction shown by the arrow in formula I, is -CR³=CR³-NR⁴- and R⁴ is hydrogen.

4. The compound of claim 1 wherein R¹R²N is 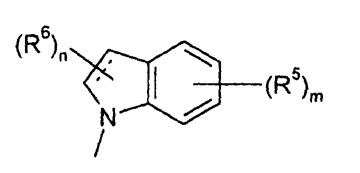 and R⁵, R⁶, m and n are as defined above.

5. The compound of claim 4 wherein each R⁵ is independently selected from 4-hydroxy, 4-amino, 5-fluoro, 5-hydroxy, 5-amino, 6-halo, 6-methyl, 6-ethenyl, 6-ethynyl, 6-nitro and 7-methyl and each R⁶ is independently selected from hydroxy, amino, N-mono- or N,N-di(C₁-C₄)alkylamino, sulfo, or (C₁-C₄)alkoxy (provided that such groups are not attached to a ring carbon which is directly adjacent to the ring N-); or R⁶ for each occurrence is independently carboxy, hydroxy(C₁-C₄)alkyl, (C₁-C₄)alkoxy(C₁-C₄)alkyl, amino(C₁-C₄)alkyl, mono-N- or di-N,N-(C₁-C₄)alkylamino(C₁-C₄)alkyl, morpholino (C₁-C₄)alkyl, carboxy(C₁-C₄)alkyl, (C₁-C₄)alkoxycarbonyl, sulfo(C₁-C₄)alkyl, and (C₁-C₄)alkyl.

6. The compound of claim 1 wherein R¹ is H and R² is (R⁵)ₘ-substituted phenyl wherein R⁵ and m are as defined above.

7. The compound of claim 6 wherein each R⁵ is independently selected from 4-fluoro-3-chloro, 3-trifluoromethyl, 4-fluoro-3-trifluoromethyl, 3-nitro-4-chloro, 3-nitro-4-fluoro, 4-fluoro-3-bromo, 3-iodo-5-amino, 3-methyl-4-fluoro, 4-amino, 3-fluoro, 3-hydroxy, 3-amino, 3-halo, 3-methyl, 3-ethenyl, 3-ethynyl, 3-nitro and 4-methyl.

8. The compound of claim 1 wherein R¹ is H and R² is Q.

9. The compound of claim 8 wherein Q is selected from pyrrolo 1,2,3,5-tetrahydro-pyrrolo[2,3-f]indole,4-,5-,6- indolyl, 1H-benzimidazol-4-yl, 1 H-benzimidazol-5-yl, 1H-indazol-4-yl, 1H-indazol-5-yl, 1H-indazol-6-yl, 1H-indazol-7-yl, 1 H-benzotriazol-4-yl, 1H-benzotriazol-5-yl, 1H-benzotriazol-6-yl, 5- or 6-benzoxazolyl, 5- or 6-benzothiazolyl, benzo[c][2,1,3]thiadiazol-4-yl, 2-, 3-, 4-, 5-, 6-, 7- or 8-quinolyl 1-, 3-, 4-, 5-, 6-, 7- or 8-isoquinolyl, 4-, 5-, 6-, 7- or 8-cinnolinyl, 5-, 6-, 7- or 8-quinazolinyl, or 2-, 5-, or 6- quinoxalinyl, which may optionally bear one or two substituents selected from fluoro, bromo, chloro, methyl ethyl, ethynyl and methoxy.

10. The compound of claim 9 wherein Q is selected from pyrrolo 5-indolyl, 1H-indazol-5-yl, 1H-benzotriazol-5-yl, 6-benzothiazolyl, benzo[c][2,1,3]thiadiazol-4-yl, 5-quinolyl, 6-quinolyl, 8-quinolyl, 5-isoquinolyl, or 5-quinoxalinyl, which may optionally bear one or two substituents selected from fluoro, bromo, chloro, methyl, ethyl, ethynyl and methoxy.

11. The compound of claim 1 selected from the group consisting of:
(3-ethynyl-phenyl)-(7H-pyrrolo[2,3-d]pyrimidin-4-yl)-amine hydrochloride;
(3-chloro-phenyl)-(7H-pyrrolo[2,3-d]pyrimidin-4-yl)-amine hydrochloride;
4-(6-chloro-2,3-dihydro-indol-1-yl)-7H-pyrrolo[2,3-d]pyrimidine hydrochloride;
(7H-pyrrolo[2,3-d]pyrimidin-4-yl)-m-tolyl-amine hydrochloride;
(1H-indol-5-yl)-(7H-pyrrolo[2,3-d]pyrimidin-4-yl)-amine hydrochloride;
(6-methylindolin-1-yl)-(7H-pyrrolo[2,3-d]pyrimidin-4-yl)-amine;
(benzo[b]thien-5-yl)-(7H-pyrrolo[2,3-d]pyrimidin-4-yl)-amine;
(6-chloro-5-fluoroindolin-1-yl)-(7H-pyrrolo[2,3-d]pyrimidin-4-yl)-amine;
(1H-indazol-5-yl)-(7H-pyrrolo[2,3-d]pyrimidin-4-yl)-amine;
1-(4-m-tolylamino-pyrrolo [2,3-d]pyrimidin-7-yl)-ethanone hydrochloride;
(5-iodo-7H-pyrrolo [2,3-d]pyrimidin-4-yl)-m-tolyl-amine;
4-(6-chloro-2,3-dihydro-indol-1-yl)-5H-pyrrolo[3,2-d]pyrimidin-6-ol;
3-ethynyl-phenyl)-[7-(2-methoxy-ethyl)-7H-pyrrolo[2,3-d]pyrimidin-4-yl]-amine;
3-ethynyl-phenyl)-{7-[2-(2-methoxy-ethoxy)-ethyl]-7H-pyrrolo[2,3-d]pyrimidin-4-yl}-amine;
(7-allyl-pyrrolo[2,3-d]pyrimidin-4-yl)-(3-ethynyl-phenyl)-amine hydrochloride;
(3-ethynyl-phenyl)-(7-methyl-pyrrolo[2,3-d]pyrimidin-4-yl)amine hydrochloride;
(5-bromo-7H-pyrrolo[2,3-d]pyrimidin-4-yl)-(3-ethynyl-phenyl)-amine;
(3-ethynyl-phenyl)-(5-iodo-7H-pyrrolo[2,3-d]pyrimidin-4-yl)-amine;
4-(3-ethynyl-phenylamino)-7H-pyrrolo[2,3-d]pyrimidine-5-carboxylic acid;
(3-ethynyl-phenyl)-(5-methyl-7H-pyrrolo[2,3-d]pyrimidin-4-yl)-amine hydrochloride;
N-(5-iodo-7H-pyrrolo[2,3-d]pyrimidin-4-yl)-N-m-tolyl-acetamide;
4-(3-ethynyl-phenylamino)-7H-pyrrolo[2,3-d]pyrimidine-5-carboxylic acid methyl ester hydrochloride;
3(-ethynyl-phenyl)-(5-methylsulfanyl-7H-pyrrolo[2,3-d]pyrimidin-4-yl)-amine; and
(3-ethynyl-phenylamino)-(7H-pyrrolo[2,3-d]pyrimidin-5-yl-carbonitrile.

12. The compound according to claim 4 selected from the group consisting of
(3-ethynyl-phenyl)-(7H-pyrrolo[2,3-d]pyrimidin-4-yl)-amine;
(3-chloro-phenyl)-(7H-pyrrolo[2,3-d]pyrimidin-4-yl)-amine;
(3-ethynyl-phenyl)-(5-methyl-7H-pyrrolo[2,3-d]pyrimidin-4-yl)amine;
4-(3-ethynyl-phenylamino)-7H-pyrrolo[2,3-d]pyrimidine-5-carboxylic acid methyl ester;
4-(3-ethynyl-phenylamino)-7H-pyrrolo[2,3-d]pyrimidine-5-carbonitrile;
(3-ethynyl-phenyl)-(5-methylsulfanyl-7H-pyrrolo[2,3-d]pyrimidin-4-yl)-amine;
(1H-indol-5-yl)-(7H-pyrrolo[2,3-d]pyrimidin-4-yl)-amine;
(5-bromo-7H-pyrrolo[2,3-d]pyrimidin-4-yl)-(3-ethynyl-phenyl)-amine; and
(3-ethynyl-phenylamino)-(7H-pyrrolo[2,3-d]pyrimidin-5-yl-carbonitrile.

13. A pharmaceutical composition for the treatment of hyperproliferative disorder in a mammal which comprises a hyperproliferative disease treating amount of a compound of claim 1 and a pharmaceutically acceptable carrier.

14. A compound according to any one of claims 1 to 12 for use in medicine.

15. The use of a compound according to any one of claims 1 to 12 in the manufacture of a medicament for the treatment of a hyperproliferative disorder.

## Patentansprüche

1. Verbindung der Formel 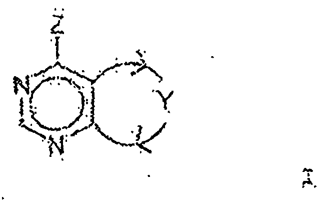 und Stereoisomere und pharmazeutisch annehmbare Salze davon, worin Y in der Richtung, die in Formel I mit dem Pfeil gezeigt ist, ausgewählt ist aus CR³=CR³-NR⁴- und -NR⁴-CR³=CR³-;
Z NR¹R² ist, worin R¹ H ist und R² mit (R⁵)ₘ substituiertes Phenyl oder Q ist oder R¹R²N eine Gruppe 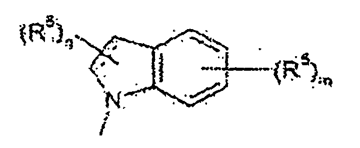 der Formel
ist, worin die gepunktete Linie eine fakultative Doppelbindung bedeutet;
jeder Rest R³ an ein Kohlenstoffatom von Y gebunden ist und unabhängig ausgewählt ist aus
a. Wasserstoff, Trifluormethyl, Halogen, Nitro, Hydroxy, Amino, Cyano, (C₁-C₄)-Alkyl, (C₁-C₄)-Alkoxy, (C₁-C₄)-Alkoxycarbonyl, (C₁-C₄)-Alkanoyloxy, (C₁-C₄)-Alkanoylamino, Carboxy, Phenoxy, Benzoyloxy, Carbamoyl, Mono-N-oder Di-N,N-di-(C₁-C₄)-alkylcarbamoyl, Mono-N- oder Di-N,N-(C₁-C₄)-alkylamino, Mono-N- oder Di-N,N-(hydroxy(C₂-C₄)-alkyl)amino, Mono-N- oder Di-N,N-((C₁-C₄)-alkoxy-(C₂-C₄)-alkyl)amino oder solchen Gruppen, die an (C₁-C₄)-Alkyl substituiert sind;
b. Hydroxy- (C₂-C₄)-alkoxy-(C₁-C₄)-alkyl, (C₁-C₄)-Alkoxy-(C₁-C₄)-alkoxy-(C₁-C₄)-alkyl, Hydroxy- (C₂-C₄)-alkylthio-(C₁-C₄), (C₁-C₄)-Alkoxy-(C₂-C₄)-alkylthio-(C₁-C₄)-alkyl, Hydroxyamino, Benzoylamino, Mono-N- oder Di-N,N-(C₁-C₄)-alkylcarbamoylmethylamino, Carbamoylmethylamino, (C₁-C₄)-Alkoxycarbonylamino, (C₁-C₄)-Alkanoylamino, Carboxymethylamino, (C₁-C₄)-Alkoxycarbonylmethylamino, (C₁-C₄)-Alkoxyamino, (C₂-C₄)-Alkanoyloxyamino, (C₁-C₄)-Alkylsulfonylamino, Ureido, (C₁-C₄)-Alkoxy-(C₁-C₄)-alkylcarbonylamino, (C₁-C₄)-Alkylsulfinyl, (C₁-C₄)-Alkylsulfonyl, (C₁-C₄)-Alkoxy-(C₂-C₄)-alkylthio, Mono-, Di- oder Trifluormethyloxy, (C₁-C₄)-Alkylendioxy, Guanidino, Aminocarbonyl, Mono-N- oder Di-N,N-(C₁-C₄)-alkylaminocarbonyl, Carboxymethoxy, (C₁-C₄)-Alkoxycarbonylmethoxy, Carbamoylmethoxy, Mono-N- oder Di-N,N-(C₁-C₄)-alkylcarbamoylmethoxy, Mono-N- oder Di-N,N- (hydroxy- (C₂-C₄)-alkyl)-carboxamido, Mono-N- oder Di-N,N-((C₁-C₄)-alkoxy-(C₂-C₄)-alkyl)carboxamido oder Bis-((C₁-C₄)-alkansulfonyl)amido oder
c. (C₂-C₄)-Alkoxy, (C₂-C₄)-Alkylthio, (C₂-C₄)-Alkanoyloxy, (C₂-C₄)-Alkylamino, (C₁-C₄)-Alkyl- (C₁-C₄)-alkylendioxy, (C₂-C₄)-Alkanoylamino, (C₂-C₄)-Alkenyl oder (C₂-C₄)-Alkinyl, jeder dieser Gruppen substituiert mit Amino, Halogen, Hydroxy, (C₂-C₄)-Alkanoyloxy, (C₁-C₄)-Alkoxy, Mono-N- oder Di-N,N-(C₁-C₄)-alkylamino, Mono-N- oder Di-N,N-(hydroxy)-(C₂-C₄)-alkyl)amino, Mono-N- oder Di-N,N-( (C₁-C₄)-alkoxy-(C₂-C₄)-alkoxy-(C₂-C₄)-alkyl)amino, (C₁-C₄)-Alkanoylamino, Carboxy- (C₁-C₄)-alkylthio-(C₄)-alkoxy, Carboxy, (C₁-C₄)-Alkoxycarbonyl, Carbamoyl, Mono-N- oder Di-N,N-(C₁-C₄)-alkylcarbamoyl, Carboxamido, Mono-N- oder Di-N,N-(C₁-C₄)-alkylcarboxamido oder Mono-N- oder Di-N,N-(hydroxy- (C₂-C₄)-alkyl)carboxamido und wobei jeder Phenylanteil in einem Substituenten R³ gegebenenfalls mit Halogen, Nitro, Trifluormethyl, Hydroxy, (C₁-C₄)-Alkoxy, (C₁-C₄)-Alkyl, Amino, Mono-N-alkylamino oder N,N-Dialkylamino mono- oder disubstituiert sein kann;
R⁴ an das N-Atom von Y gebunden ist und ausgewählt ist aus Wasserstoff, (C₁-C₄)-Alkyl, (C₁-C₄)-Alkoxycarbonyl, (C₁-C₄)-Alkanoyl, (C₁-C₄)-Alkylsulfonyl, Arylsulfonyl, Allyl oder einer (C₂-C₄)-Alkyl-, (C₂-C₄)-Alkanoyl- oder (C₂-C₄) -Alkoxycarbonyl-, (C₂-C₄)-Alkylsulfonylgruppe, jeder dieser Gruppen substituiert mit Amino, Halogen, Hydroxy, (C₂-C₄)-Alkanoyloxy, (C₁-C₄)-Alkoxy, Mono-N- oder Di-N,N-(C₁-C₄)-alkylamino, Mono-N- oder Di-N,N-(hydroxy-(C₂-C₄)-alkylamino, Mono-N- oder Di-N,N-((C₁-C₄)-alkoxy-(C₂-C₄)-alkyl)amino, (C₁-C₄)-Alkanoylamino, Carboxy, (C₁-C₄)-Alkoxycarbonyl, Carbamoyl, Mono-N- oder Di-N,N- (C₁-C₄)-alkylcarbamoyl, Carboxamido, Mono-N- oder Di-N,N-(C₁-C₄)-alkylcarboxamido oder Mono-N- oder Di-N,N-(hydroxy-(C₂-C₄)-alkyl)carboxamido;
jeder Rest R⁵ unabhängig ausgewählt ist aus Mono-, Dioder Trifluormethyl, Halogen, Nitro, Hydroxy, Amino, Azido, Isothiocyano, (C₁-C₄)-Alkyl, (C₁-C₄)-Alkoxy, (C₂-C₆)-Alkenyl, (C₂-C₆)-Alkinyl, (C₁-C₄)-Alkylendioxy, Cyano, Trifluormethylcarbonylamino, (C₁-C₄)-Alkanoylamino, (C₁-C₄)-Alkanoyl, N-Mono- oder N,N-Di-(C₁-C₄)-alkylamino, (C₁-C₄)-Alkylsulfonylamino, Trifluormethylsulfonylamino, (C₁-C₄)-Alkylthio, (C₁-C₄)-Alkylsulfinyl oder (C₁-C₄)-Alkylsulfonyl und der (C₁-C₄)-Alkylendioxyrest an beiden Enden an benachbarte Kohlenstoffatome an der Benzoleinheit gebunden ist;
jeder Rest R⁶ unabhängig ausgewählt ist aus Hydroxy, Amino, N-Mono- oder N,N-Di-(C₁-C₄)-alkylamino, Sulfo oder (C₁-C₄)-Alkoxy (mit dem Vorbehalt, dass diese Gruppen nicht an ein Ringkohlenstoffatom gebunden sind, das direkt dem Ring-N- benachbart ist) oder R⁶ bei jedem Vorkommen unabhängig Carboxy, Hydroxy-(C₁-C₄)-alkyl, (C₁-C₄)-Alkoxy-(C₁-C₄)-alkyl, Amino-(C₁-C₄)-alkyl, Mono-N-oder Di-N,N-(C₁-C₄)-alkylamino-(C₁-C₄)-alkyl, Morpholino-(C₁-C₄)-alkyl, 4- (C₁-C₄)-Alkylpiperazin-1-yl-(C₁-C₄)-alkyl, Carboxy-(C₁-C₄)-alkyl, (C₁-C₄)-Alkoxycarbonyl, Sulfo- (C₁-C₄)-alkyl, Pyridyl-(C₁-C₄)-alkyl oder (C₁-C₄)-Alkyl ist;
m eine ganze Zahl von 1 bis 3 ist;
n 0, 1 oder 2 ist;
p 0 oder eine ganze Zahl von 1 bis 3 ist;
Q ein 9- oder 10-gliedriger bicyclischer heteroarylcyclischer Anteil ist oder ein hydriertes Derivat davon, der/das ein oder zwei Stickstoffheteroatome enthält und gegebenenfalls ein weiteres Heteroatom enthält ausgewählt aus Stickstoff, Sauerstoff und Schwefel, oder Q ein 9- oder 10-gliedriger bicyclischer Arylanteil ist oder ein hydriertes Derivat davon, wobei der heterocyclische oder Arylanteil oder die hydrierten Derivate davon gegebenenfalls ein oder zwei Substituenten tragen können ausgewählt aus Halogeno, Hydroxy, Oxo, Amino, Nitro, Carbamoyl, (C₁-C₄)-Alkyl, (C₁-C₄)-Alkoxy, (C₁-C₄)-Alkylamino, Di- [(C₁-C₄)-alkyl]amino, (C₂-C₄)-Alkanoylamino und (C₂-C₄)-Alkinyl, mit dem Vorbehalt, dass dann, wenn Y in der Richtung, die durch den Pfeil in Formel I gezeigt ist, -NR⁴-CR³=CR³- ist, R³ = CH₃ ist und R⁴ = H ist, dass dann R⁵ nicht 4-CH₃, 3,5-(CH₃)₂, 2,6-(CH₃)₂, 2-C₂H₅, 4-C₂H₅, 4-n-C₄H₉, 2-Cl, 4-Cl, 3,4-Cl₂, 2-F oder 3-CF₃ ist.

2. Verbindung nach Anspruch 1, worin jeder Rest R³ unabhängig ausgewählt ist aus Wasserstoff, Hydroxy, (C₁-C₄)-Alkoxy, Hydroxy- (C₂-C₄)-alkoxy, Amino- (C₂-C₄)-alkyl, Amino- (C₂-C₄) -alkoxy, (C₁-C₄)-Alkoxy- (C₂-C₄)-alkoxy, Hydroxy-(C₁-C₄)-alkyl-(C₁-C₄)-alkylendioxy, (C₁-C₄)-Alkoxy-(C₁-C₄)-alkyl- (C₁-C₄)-alkylendioxy, Mono-N- oder Di-N,N-(C₁-C₄)-alkylamino- (C₂-C₄)-alkoxy, 3- oder 4-(C₁-C₄)-Alkoxy-(2-hydroxy) - (C₃-C₄) -alkoxy, Carboxy- (C₁-C₄)-alkoxy, (C₁-C₄)-Alkoxy-(C₁-C₄)-alkanoyloxy, Nitro, Hydroxylamino, Amino, Mono-N- oder Di-N,N-(C₁-C₄)-alkylamino, (C₁-C₄)-Alkanoylamino, Hydroxy-(C₂-C₄)-alkylamino, (C₁-C₄)-Alkoxy- (C₂-C₄)-alkylamino, (C₁-C₄)-Alkylsulfonamido, Bis-(C₁-C₄)-alkansulfonamido, Di-N,N-(C₁-C₄)-alkylamino-(C₂-C₄)-alkylamino, (C₁-C₄)-Alkylamino-(C₂-C₄)-alkylamino, (C₁-C₄)-Alkoxy-(C₁-C₄)-alkylcarbonylamino, Carboxy, (C₁-C₄)-Alkoxycarbonyl, (C₁-C₄)-Alkoxycarbonyl-(C₁-C₄)-alkoxy, Amido, Mono-N- oder Di-N,N-(C₁-C₄)-alkylaminocarbonyl, Mono-N- oder Di-N,N-(hydroxy-(C₂-C₄)-alkyl)aminocarbonyl, (C₁-C₄)-Alkyl, Hydroxy- (C₁-C₄)-alkyl, Mono-N- oder Di-N,N-((C₁-C₄)-alkoxy-(C₁-C₄)-alkyl)amino-(C₁-C₄)-alkyl, Mono-N- oder Di-N,N-(C₁-C₄)-alkylamino-(C₁-C₄)-alkyl, (C₁-C₄)-Alkanoylamino- (C₁-C₄)-alkyl, (C₁-C₄)-Alkoxy-(C₂-C₄)-alkoxy-(C₁-C₄)-alkyl, (C₁-C₄)-Alkylthio, (C₁-C₄)-Alkoxy-(C₂-C₄)-alkylthio oder Hydroxy-(C₂-C₄)-alkylthio und R⁴ ausgewählt ist aus Wasserstoff, (C₂-C₄)-Alkyl, Hydroxy-(C₂-C₄)-alkyl oder Hydroxy- (C₂-C₄)-alkyl, Amino-(C₂-C₆)-alkyl, (C₂-C₄)-Alkoxycarbonyl, wobei jede dieser Gruppen substituiert ist mit Amino, Halogen, Hydroxy, (C₂-C₄)-Alkanoyloxy, (C₁-C₄)-Alkoxy, Mono-N- oder Di-N,N-(C₁-C₄)-alkylamino, Mono-N- oder Di-N,N- (hydroxy- (C₂-C₄)-alkyl)amino, Mono-N- oder Di-N,N-((C₁-C₄)-alkoxy-(C₂-C₄)-alkyl)amino, Sulfonylaryl-(C₁-C₄)-alkylamin, (C₁-C₄)-Alkanoylamino, Carboxy, (C₁-C₄)-Alkoxycarbonyl, Carbamoyl, Mono-N- oder Di-N,N-(C₁-C₄)-Alkylcarbamoyl, Carboxamido, Mono-N- oder Di-N,N-(C₁-C₄)-alkylcarboxamido oder Mono-N- oder Di-N,N-(hydroxy-(C₂-C₄)-alkyl)carboxamido.

3. Verbindung nach Anspruch 2, wobei Y in der in der Formel I durch den Pfeil gezeigten Richtung -CR³=CR³-NR⁴- ist und R⁴ Wasserstoff ist.

4. Verbindung nach Anspruch 1, worin R¹R²N 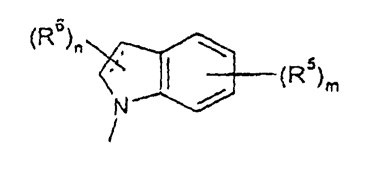 ist und R⁵, R⁶, m und n wie oben definiert sind.

5. Verbindung nach Anspruch 4, wobei jeder Rest R⁵ unabhängig ausgewählt ist aus 4-Hydroxy, 4-Amino, 5-Fluor, 5-Hydroxy, 5-Amino, 6-Halogen, 6-Methyl, 6-Ethenyl, 6-Ethinyl, 6-Nitro und 7-Methyl und jeder Rest R⁶ unabhängig ausgewählt ist aus Hydroxy, Amino, N-Mono- oder N,N-Di-(C₁-C₄)-alkylamino, Sulfo oder (C₁-C₄)-Alkoxy (mit dem Vorbehalt, dass solche Gruppen nicht an ein Ringatom gebunden sind, das direkt dem Ring N- benachbart ist) oder R⁶ bei jedem Vorkommen unabhängig Carboxy, Hydroxy-(C₁-C₄)-alkyl, (C₁-C₄)-Alkoxy-(C₁-C₄)-alkyl, Amino- (C₁-C₄)-alkyl, Mono-N- oder Di-N,N- (C₁-C₄)-alkylamino-(C₁-C₄)-alkyl, Morpholino-(C₁-C₄)-alkyl, Carboxy-(C₁-C₄)-alkyl, (C₁-C₄)-Alkoxycarbonyl, Sulfo-(C₁-C₄)-alkyl und (C₁-C₄)-Alkyl ist.

6. Verbindung nach Anspruch 1, worin R¹ H ist und R² (R⁵)ₘsubstituiertes Phenyl ist, worin R⁵ und m wie oben definiert sind.

7. Verbindung nach Anspruch 6, worin jeder Rest R⁵ unabhängig ausgewählt ist aus 4-Fluor-3-chlor, 3-Trifluormethyl, 4-Fluor-3-trifluormethyl, 3-Nitro-4-chlor, 3-Nitro-4-fluor, 4-Fluor-3-brom, 3-Iod-5-amino, 3-Methyl-4-fluor, 4-Amino, 3-Fluor, 3-Hydroxy, 3-Amino, 3-Halogen, 3-Methyl, 3-Ethenyl, 3-Ethinyl, 3-Nitro und 4-Methyl.

8. Verbindung nach Anspruch 1, worin R¹ H ist und R² Q ist.

9. Verbindung nach Anspruch 8, worin Q ausgewählt ist aus Pyrrolo-1,2,3,5-tetrahydropyrrolo[2,3-f]indol, 4,5,6-Indolyl, 1H-Benzimidazol-4-yl, 1H-Benzimidazol-5-yl, 1H-Indazol-4-yl, 1H-Indazol-5-yl, 1H-Indazol-6-yl, 1H-Indazol-7-yl, 1H-Benzotriazol-4-yl, 1H-Benzotriazol-5-yl, 1H-Benzotriazol-6-yl, 5- oder 6-Benzoxazolyl, 5-oder 6-Benzothiazolyl, Benzo[c][2,1,3]thiadiazol-4-yl, 2-, 3-, 4-, 5-, 6-, 7- oder 8-Chinolyl, 1-, 3-, 4-, 5-, 6-, 7- oder 8-Isochinolyl, 4-, 5-, 6-, 7- oder 8-Cinnolinyl, 5-, 6-, 7- oder 8-Chinazolinyl oder 2-, 5-oder 6-Chinoxalinyl, die gegebenenfalls einen oder zwei Substituenten tragen können ausgewählt aus Fluor, Brom, Chlor, Methyl, Ethyl, Ethinyl und Methoxy.

10. Verbindung nach Anspruch 9, worin Q ausgewählt ist aus Pyrrolo-5-indolyl, 1H-Indazol-5-yl, 1H-Benzotriazol-5-yl, 6-Benzothiazolyl, Benzo[c][2,1,3]thiadiazol-4-yl, 5-Chinolyl, 6-Chinolyl, 8-Chinolyl, 5-Isochinolyl oder 5-Chinoxalinyl, die gegebenenfalls einen oder zwei Substituenten tragen können ausgewählt aus Fluor, Brom, Chlor, Methyl, Ethyl, Ethinyl und Methoxy.

11. Verbindung nach Anspruch 1, ausgewählt aus der Gruppe bestehend aus
(3-Ethinylphenyl)-(7H-pyrrolo[2,3-d]pyrimidin-4-yl) aminhydrochlorid;
(3-Chlorphenyl)-(7H-pyrrolo[2,3-d]pyrimidin-4-yl)aminhydrochlorid ;
(4- (6-Chlor-2,3-dihydroindol-1-yl)-7H-pyrrolo[2,3-d]pyrimidinhydrochlorid;
(7H-pyrrolo [2,3-d]pyrimidin-4-yl)-m-tolylaminhydrochlorid;
(1H-Indol-5-yl)-(7H-pyrrolo[2,3-d]pyrimidin-4-yl)-aminhydrochlorid;
(6-Methylindolin-1-yl)-(7H-pyrrolo[2,3-d]pyrimidin-4-yl) amin;
(Benzo[b]thien-5-yl)- (7H-pyrrolo[2,3-d]pyrimidin-4-yl)amin;
(6-Chlor-5-fluorindolin-1-yl)-(7H-pyrrolo[2,3-d]pyrimidin-4-yl)amin;
(1H-Indazol-5-yl)-(7H-pyrrolo[2,3-d]pyrimidin-4-yl)amin;
1-(4-m-Tolylaminopyrrolo[2,3-d]pyrimidin-7-yl)ethanonhydrochlorid;
(5-Iod-7H-pyrrolo[2,3-d]pyrimidin-4-yl)-m-tolylamin;
4-(6-Chlor-2,3-dihydroindol-1-yl)-5H-pyrrolo[3,2-d]pyrimidin-6-ol;
(3-Ethinylphenyl)-[7-(2-methoxyethyl)-7H-pyrrolo[2,3-d]pyrimidin-4-yl]amin;
(3-Ethinylphenyl)-{7-[2-(2-methoxyethoxy)ethyl]-7Hpyrrolo[2,3-d]pyrimidin-4-yl}amin;
(7-Allylpyrrolo[2,3-d]pyrimidin-4-yl)-(3-ethinylphenyl)-aminhydrochlorid;
(3-Ethinylphenyl)-(7-methylpyrrolo[2,3-d]pyrimidin-4-yl)aminhydrochlorid;
(5-Brom-7H-pyrrolo[2,3-d]pyrimidin-4-yl)-(3-ethinylphenyl)amin;
(3-Ethinylphenyl)-(5-iod-7H-pyrrolo[2,3-d]pyrimidin-4-yl)amin;
4- (3-Ethinylphenylamino)-7H-pyrrolo [2,3-d]pyrimidin-5-carbonsäure;
(3-Ethinylphenyl)-(5-methyl-7H-pyrrolo[2,3-d]pyrimidin-4-yl)aminhydrochlorid;
N- (5-Iod-7H-pyrrolo[2,3-d]pyrimidin-4-yl)-N-m-tolylacetamid;
4- (3-Ethinylphenylamino)-7H-pyrrolo[2,3-d]pyrimidin-5-carbonsäure-methylester-hydrochlorid;
(3-Ethinylphenyl)-(5-methylsulfanyl-7H-pyrrolo [2,3-d]-pyrimidin-4-yl)amin und
(3-Ethinylphenylamino)-(7H-pyrrolo [2,3-d]pyrimidin-5-yl)carbonitril.

12. Verbindung nach Anspruch 4, ausgewählt aus der Gruppe bestehend aus
(3-Ethinylphenyl) - (7H-pyrrolo[2,3-d]pyrimidin-4-yl)amin;
(3-Chlorphenyl) - (7H-pyrrolo[2,3-d]pyrimidin-4-yl)amin;
(3-Ethinylphenyl) - (5-methyl-7H-pyrrolo[2,3-d]pyrimidin-4-yl)amin;
4-(3-Ethinylphenylamino)-7H-pyrrolo[2,3-d]pyrimidin-5-carbonsäuremethylester;
4-(3-Ethinylphenylamino)-7H-pyrrolo[2,3-d]pyrimidin-5-carbonitril;
(3-Ethinylphenyl)-(5-methylsulfanyl-7H-pyrrolo[2,3-d]pyrimidin-4-yl)amin;
(1H-Indol-5-yl)-(7H-pyrrolo[2,3-d]pyrimidin-4-yl)-amin;
(5-Brom-7H-pyrrolo[2,3-d]pyrimidin-4-yl)-(3-ethinylphenyl)amin und
(3-Ethinylphenylamino)-(7H-pyrrolo[2,3-d]pyrimidin-5-ylcarbonitril.

13. Pharmazeutische Zusammensetzung zur Behandlung eines hyperproliferativen Leidens bei einem Säugetier, die eine das hyperproliferative Leiden behandelnde Menge einer Verbindung nach Anspruch 1 und einen pharmazeutisch annehmbaren Träger enthält.

14. Verbindung nach einem der Ansprüche 1 bis 12 zur Verwendung als Arzneimittel.

15. Verwendung einer Verbindung nach einem der Ansprüche 1 bis 12 zur Herstellung eines Arzneimittels zur Behandlung eines hyperproliferativen Leidens.

## Revendications

1. Composé de formule 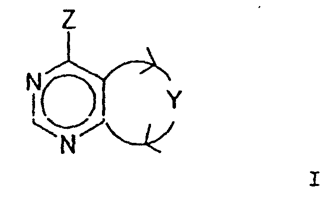 et ses stéréo-isomères, ainsi que ses sels pharmaceutiquement acceptables, formule dans laquelle Y, dans le sens indiqué par la flèche dans la formule I, est choisi entre des groupes CR³=CR³-NR⁴- et -NR⁴-CR³=CR³- ;
Z représente un groupe NR¹R² dans lequel R¹ représente H et R² représente un groupe phényle substitué avec un substituant (R⁵)ₘ ou Q ou bien R¹R²N représente un groupe de formule 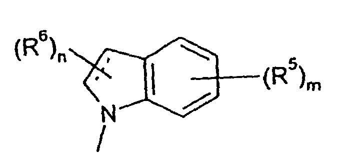 dans lequel la ligne discontinue représente une double liaison facultative ;
chaque groupe R³ est fixé à un atome de carbone dans Y et est choisi, indépendamment, entre
a. un atome d'hydrogène, des groupes trifluorométhyle, halogéno, nitro, hydroxy, amino, cyano, alkyle en C₁ à C₄, alkoxy en C₁ à C₄, (alkoxy en C₁ à C₄)carbonyle, alcanoyloxy en C₁ à C₄, alcanoylamino en C₁ à C₄, carboxy, phénoxy, benzoyloxy, carbamoyle, mono-N- ou di-N,N-di(alkyle en C₁ à C₄)carbamoyle, mono-N- ou di-N,N-(alkyle en C₁ à C₄)amino, mono-N ou di-N,N-(hydroxy(alkyle en C₂ à C₄))amino, mono-N ou di-N,N-((alkoxy en C₁ à C₄)(alkyle en C₂ à C₄))amino, ou bien de tels groupes substitués sur le groupe alkyle en C₁ à C₄ ;
b. des groupes hydroxy(alkoxy en C₂ à C₄)(alkyle en C₁ à C₄), (alkoxy en C₁ à C₄)(alkoxy en C₁ à C₄)(alkyle en C₁ à C₄), hydroxy(alkyle en C₂ à C₄)thio(alkyle en C₁ à C₄), (alkoxy en C₁ à C₄)(alkyle en C₂ à C₄)thio(alkyle en C₁ à C₄), hydroxyamino, benzoylamino, mono-N- ou di-N,N-(alkyle en C₁ à C₄)carbamoylméthylamino, carbamoylméthylamino, (alkoxy en C₁ à C₄)carbonylamino, alcanoylamino en C₁ à C₄, carboxyméthylamino, (alkoxy en C₁ à C₄)carbonylméthylamino, alkoxyamino en C₁ à C₄, (alcanoyle en C₂ à C₄)oxyamino, (alkyle en C₁ à C₄)sulfonylamino, uréido, (alkoxy en C₁ à C₄) (alkyle en C₁ à C₄)carbonylamino, alkylsulfinyle en C₁ à C₄, alkylsulfonyle en C₁ à C₄, (alkoxy en C₁ à C₄) (alkyle en C₂ à C₄)thio, mono-, di- ou tri-fluorométhyloxy, alkylènedioxy en C₁ à C₄, guanidino, aminocarbonyle, mono-N ou di-N,N-(alkyle en C₁ à C₄)aminocarbonyle, carboxyméthoxy, (alkoxy en C₁ à C₄)carbonylméthoxy, carbamoylméthoxy, mono-N ou di-N,N-(alkyle en C₁ à C₄)carbamoylméthoxy, mono-N ou di-N,N-(hydroxyalkyle en C₂ à C₄)carboxamido, mono-N ou di-N,N-((alkoxy en C₁ à C₄)(alkyle en C₂ à C₄))carboxamido ou bis(alcanesulfonyle en C₁ à C₄)amido ; ou
c. de groupes alkoxy en C₂ à C₄, alkylthio en C₂ à C₄, alcanoyloxy en C₂ à C₄, alkylamino en C₂ à C₄, (alkyle en C₁ à C₄) (alkylène en C₁ à C₄)dioxy, alcanoylamino en C₂ à C₄, alcényle en C₂ à C₄ ou alcynyle en C₂ à C₄ ; chacun de ces groupes substitué avec des substituants amino, halogéno, hydroxy, alcanoyloxy en C₂ à C₄, alkoxy en C₁ à C₄, mono-N ou di-N,N-(alkyle en C₁ à C₄)amino, mono-N ou di-N,N-(hydroxyalkyle en C₂ à C₄)amino, mono-N ou di-N,N-((alkoxy en C₁ à C₄)(alkoxy en C₂ à C₄)(alkyle en C₂ à C₄))amino, alcanoylamino en C₁ à C₄, carboxy(alkyle en C₁ à C₄)thio(alkoxy en C₄), carboxy, (alkoxy en C₁ à C₄)carbonyle, carbamoyle, mono-N ou di-N,N-(alkyle en C₁ à C₄)carbamoyle, carboxamido, mono-N ou di-N,N-(alkyle en C₁ à C₄)carboxamido ou mono-N ou di-N,N-(hydroxyalkyle en C₂ à C₄)carboxamido ; et n'importe quel groupe phényle dans un substituant R³ est facultativement mono- ou disubstitué avec un substituant halogéno, nitro, trifluorométhyle, hydroxy, alkoxy en C₁ à C₄, alkyle en C₁ à C₄, amino, mono-N, alkylamino ou N,N-dialkylamino ;
R⁴ est fixé à l'atome de N dans Y et est choisi entre : un atome d'hydrogène, des groupes alkyle en C₁ à C₄, (alkoxy en C₁ à C₄)carbonyle, alcanoyle en C₁ à C₄, alkylsulfonyle en C₁ à C₄, arylsulfonyle, allyle ; ou un groupe alkyle en C₂ à C₄, alcanoyle en C₂ à C₄, (alkoxy en C₂ à C₄)carbonyle ou alkylsulfonyle en C₂ à C₄, chacun de ces groupes étant substitué avec des substituants amino, halogéno, hydroxy, alcanoyloxy en C₂ à C₄, alkoxy en C₁ à C₄, mono-N ou di-N,N-(alkyle en C₁ à C₄)amino, mono-N ou di-N,N-(hydroxyalkyle en C₂ à C₄)amino, mono-N ou di-N,N-((alkoxy en C₁ à C₄)(alkyle en C₂ à C₄))amino, alcanoylamino en C₁ à C₄, carboxy, (alkoxy en C₁ à C₄)carbonyle, carbamoyle, mono-N ou di-N,N-(alkyle en C₁ à C₄)carbamoyle, carboxamido, mono-N ou di-N,N-(alkyle en C₁ à C₄)-carboxamido ou mono-N ou di-N,N-(hydroxyalkyle en C₂ à C₄)carboxamido ;
chaque groupe R⁵ est choisi, indépendamment, entre des groupes mono-, di- ou trifluorométhyle, halogéno, nitro, hydroxy, amino, azido, isothiocyano, alkyle en C₁ à C₄, alkoxy en C₁ à C₄, alcényle en C₂ à C₆, alcynyle en C₂ à C₆, alkylènedioxy en C₁ à C₄, cyano, trifluorométhylcarbonylamino, alcanoylamino en C₁ à C₄, alcanoyle en C₁ à C₄, N-mono- ou N,N-di(alkyle en C₁ à C₄)amino, (alkyle en C₁ à C₄)sulfonylamino, trifluorométhylsulfonylamino, alkylthio en C₁ à C₄, alkylsulfinyle en C₁ à C₄ ou alkylsulfonyle en C₁ à C₄ et ledit groupe alkylènedioxy en C₁ à C₄ est lié aux deux extrémités à des atomes de carbone adjacents sur le groupement benzénique ;
chaque groupe R⁶ est choisi, indépendamment, entre des groupes hydroxy, amino, N-mono- ou N,N-di(alkyle en C₁ à C₄)amino, sulfo et alkoxy en C₁ à C₄ (sous réserve que ces groupes ne soient pas fixés à un atome de carbone de noyau qui est directement adjacent à l'atome N- du noyau), ou bien R⁶ à chaque emplacement représente, indépendamment, un groupe carboxy, hydroxyalkyle en C₁ à C₄, (alkoxy en C₁ à C₄) (alkyle en C₁ à C₄), aminoalkyle en C₁ à C₄, mono-N- ou di-N,N-(alkyle en C₁ à C₄)amino(alkyle en C₁ à C₄), morpholino(alkyle en C₁ à C₄), 4-(alkyle en C₁ à C₄)pipérazine-1-yl(alkyle en C₁ à C₄), carboxy(alkyle en C₁ à C₄), (alkoxy en C₁ à C₄) carbonyle, sulfoalkyle en C₁ à C₄, pyridyl(alkyle en C₁ à C₄) ou alkyle en C₁ à C₄ ;
m représente un nombre entier de 1 à 3 ;
n est égal à 0, 1 ou 2 ;
p est égal à 0 ou un nombre entier de 1 à 3 ;
Q représente un groupement cyclique hétéroaryle bicyclique nona- ou décagonal ou un de ses dérivés hydrogénés, contenant un ou deux hétéroatomes d'azote et contenant facultativement un hétéroatome supplémentaire choisi entre l'azote, l'oxygène et le soufre, ou bien Q représente un groupement aryle bicyclique nona- ou décagonal, ou un de ses dérivés hydrogénés, ce groupement hétérocyclique ou aryle ou ses dérivés hydrogénés pouvant porter facultativement un ou deux substituants choisis entre des substituants halogéno, hydroxy, oxo, amino, nitro, carbamoyle, alkyle en C₁ à C₄, alkoxy en C₁ à C₄, alkylamino en C₁ à C₄, di(alkyle en C₁ à C₄)amino, alcanoylamino en C₂ à C₄ et alcynyle en C₂ à C₄, sous réserve que, lorsque Y, dans le sens indiqué par la flèche dans la formule I, représente un groupe -NR⁴-CR³=CR³-, R³ représente un groupe CH₃ et R⁴ représente H, alors R⁵ ne représente pas un groupe 4-CH₃, 3,5-(CH₃)₂, 2,6-(CH₃)₂, 2-C₂H₅, 4-C₂H₅, 4-n-C₄H₉, 2-Cl, 4-Cl, 3,4-Cl₂, 2-F ou 3-CF₃.

2. Composé suivant la revendication 1, dans lequel chaque groupe R³ est choisi, indépendamment, entre un atome d'hydrogène, les groupes hydroxy, alkoxy en C₁ à C₄, hydroxyalkoxy en C₂ à C₄, aminoalkyle en C₂ à C₄, aminoalkoxy en C₂ à C₄, (alkoxy en C₁ à C₄)(alkoxy en C₂ à C₄), hydroxy(alkyle en C₁ à C₄) (alkylène en C₁ à C₄)dioxy, (alkoxy en C₁ à C₄) (alkyle en C₁ à C₄) (alkylène en C₁ à C₄)-dioxy, mono-N- ou di-N,N-(alkyle en C₁ à C₄)amino(alkoxy en C₂ à C₄), 3- ou 4-(alkoxy en C₁ à C₄) (2-hydroxy)(alkoxy en C₃ ou C₄), carboxy(alkoxy en C₁ à C₄), (alkoxy en C₁ à C₄)-(alcanoyle en C₁ à C₄)oxy, nitro, hydroxylamino, amino, mono-N ou di-N,N-(alkyle en C₁ à C₄)amino, alcanoylamino en C₁ à C₄, hydroxyalkylamino en C₂ à C₄, (alkoxy en C₁ à C₄)-(alkyle en C₂ à C₄)amino, alkylsulfonamido en C₁ à C₄, bis-(alcane en C₁ à C₄) sulfonamido, di-N,N-(alkyle en C₁ à C₄)-amino(alkyle en C₂ à C₄)amino, (alkyle en C₁ à C₄)amino-(alkyle en C₂ à C₄)amino, (alkoxy en C₁ à C₄) (alkyle en C₁ à C₄)carbonylamino, carboxy, (alkoxy en C₁ à C₄)carbonyle, (alkoxy en C₁ à C₄)carbonyl(alkoxy en C₁ à C₄), amido, mono-N ou di-N,N-(alkyle en C₁ à C₄)aminocarbonyle, mono-N ou di-N,N-(hydroxyalkyle en C₂ à C₄)aminocarbonyle, alkyle en C₁ à C₄, hydroxyalkyle en C₁ à C₄, mono-N ou di-N,N-((alkoxy en C₁ à C₄)(alkyle en C₁ à C₄))amino(alkyle en C₁ à C₄), mono-N ou di-N,N-(alkyle en C₁ à C₄)amino(alkyle en C₁ à C₄), (alcanoyle en C₁ à C₄)amino(alkyle en C₁ à C₄), (alkoxy en C₂ à C₄)(alkoxy en C₁ à C₄) (alkyle en C₁ à C₄), alkylthio en C₁ à C₄, (alkoxy en C₁ à C₄)(alkyle en C₂ à C₄)thio ou hydroxy(alkyle en C₂ à C₄)thio ; et R⁴ est choisi entre un atome d'hydrogène ; des groupes alkyle en C₂ à C₄, hydroxyalkyle en C₂ à C₄, aminoalkyle en C₂ à C₆ et (alkoxy en C₂ à C₄)carbonyle, chacun de ces groupes étant substitué avec des substituants amino, halogéno, hydroxy, alcanoyloxy en C₂ à C₄, alkoxy en C₁ à C₄, mono-N ou di-N,N-(alkyle en C₁ à C₄)amino, mono-N ou di-N,N-(hydroxyalkyle en C₂ à C₄)amino, mono-N ou di-N,N-((alkoxy en C₁ à C₄)(alkyle en C₂ à C₄))amino, sulfonylaryl(alkyle en C₁ à C₄)amine, alcanoylamino en C₁ à C₄, carboxy, (alkoxy en C₁ à C₄)carbonyle, carbamoyle, mono-N ou di-N,N-(alkyle en C₁ à C₄)carbamoyle, carboxamido, mono-N ou di-N,N-(alkyle en C₁ à C₄)carboxamido ou mono-N- ou di-N,N-(hydroxyalkyle en C₂ à C₄)carboxamido.

3. Composé suivant la revendication 2, dans lequel Y, dans le sens représenté par la flèche dans la formule I, représente un groupe -CR³=CR³-NR⁴- et R⁴ représente un atome d'hydrogène.

4. Composé suivant la revendication 1, dans lequel R¹R²N représente un groupe 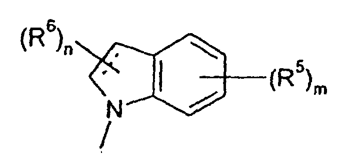 et R⁵, R⁶, m et n répondent aux définitions précitées.

5. Composé suivant la revendication 4, dans lequel chaque groupe R⁵ est choisi, indépendamment, entre des groupes 4-hydroxy, 4-amino, 5-fluoro, 5-hydroxy, 5-amino, 6-halogéno, 6-méthyle, 6-éthényle, 6-éthynyle, 6-nitro et 7-méthyle et chaque groupe R⁶ est choisi, indépendamment, entre des groupes hydroxy, amino, N-mono ou N,N-di(alkyle en C₁ à C₄)amino, sulfo ou alkoxy en C₁ à C₄ (sous réserve que ces groupes ne soient pas fixés à un atome de carbone de noyau qui est directement adjacent à l'atome N- du noyau) ; ou bien R⁶, à chaque emplacement, représente, indépendamment, un groupe carboxy, hydroxyalkyle en C₁ à C₄, (alkoxy en C₁ à C₄)(alkyle en C₁ à C₄), aminoalkyle en C₁ à C₄, mono-N- ou di-N,N-(alkyle en C₁ à C₄)amino(alkyle en C₁ à C₄), morpholino(alkyle en C₁ à C₄), carboxy(alkyle en C₁ à C₄), (alkoxy en C₁ à C₄)carbonyle, sulfoalkyle en C₁ à C₄ ou alkyle en C₁ à C₄.

6. Composé suivant la revendication 1, dans lequel R¹ représente H et R² représente un groupe phényle à substituant (R⁵)ₘ-, dans lequel R⁵ et m répondent aux définitions précitées.

7. Composé suivant la revendication 6, dans lequel chaque groupe R⁵ est choisi, indépendamment, entre des groupes 4-fluoro-3-chloro, 3-trifluorométhyle, 4-fluoro-3-trifluorométhyle, 3-nitro-4-chloro, 3-nitro-4-fluoro, 4-fluoro-3-bromo, 3-iodo-5-amino, 3-méthyl-4-fluoro, 4-amino, 3-fluoro, 3-hydroxy, 3-amino, 3-halogéno, 3-méthyle, 3-éthényle, 3-éthynyle, 3-nitro et 4-méthyle.

8. Composé suivant la revendication 1, dans lequel R¹ représente H et R² représente Q.

9. Composé suivant la revendication 8, dans lequel Q est choisi entre des groupes pyrrolo, 1,2,3,5-tétrahydropyrrolo[2,3-f]indole, 4-,5-,6-indolyle, 1H-benzimidazole-4-yle, 1H-benzimidazole-5-yle, 1H-indazole-4-yle, 1H-indazole-5-yle, 1H-indazole-6-yle, 1H-indazole-7-yle, 1H-benzotriazole-4-yle, 1H-benzotriazole-5-yle, 1H-benzotriazole-6-yle 5- ou 6-benzoxazolyle, 5- ou 6-benzothiazolyle, benzo[c][2,1,3]thiadiazole-4-yle, 2-, 3-, 4-, 5-, 6-, 7- ou 8-quinolyle, 1-, 3-, 4-, 5-, 6-, 7- ou 8-isoquinolyle, 4-, 5-, 6-, 7- ou 8-cinnolinyle, 5-, 6-, 7-ou 8-quinazolinyle et 2-, 5- ou 6-quinoxalinyle, qui peuvent porter facultativement un ou deux substituants choisis entre des substituants fluoro, bromo, chloro, méthyle, éthyle, éthynyle et méthoxy.

10. Composé suivant la revendication 9, dans lequel Q est choisi entre des groupes pyrrolo, 5-indolyle, 1H-indazole-5-yle, 1H-benzotriazole-5-yle, 6-benzothiazolyle, benzo[c][2,1,3]thiadiazole-4-yle, 5-quinoyle, 6-quinolyle, 8-quinolyle, 5-isoquinolyle ou 5-quinoxalinyle qui peuvent porter facultativement un ou deux substituants choisis entre des substituants fluoro, bromo, chloro, méthyle, éthyle, éthynyle et méthoxy.

11. Composé suivant la revendication 1, choisi dans le groupe consistant en :
chlorhydrate de (3-éthynylphényl)-(7H-pyrrolo[2,3-d]-pyrimidine-4-yl)aminé ;
chlorhydrate de (3-chlorophényl)-(7H-pyrrolo[2,3-d]-pyrimidine-4-yl)amine ;
chlorhydrate de 4-(6-chloro-2,3-dihydro-indole-1-yl)-7H-pyrrolo[2,3-d]-pyrimidine ;
chlorhydrate de (7H-pyrrolo[2,3-d]-pyrimidine-4-yl)-m-tolylamine ;
chlorhydrate de (1H-indole-5-yl)-(7H-pyrrolo[2,3-d]-pyrimidine-4-yl)aminé ;
(6-méthylindoline-1-yl)-(7H-pyrrolo[2,3-d]pyrimidine-4-yl)amine ;
(benzo[b]thién-5-yl)-(7H-pyrrolo[2,3-d]pyrimidine-4-yl)aminé ;
(6-chloro-5-fluoro-indoline-1-yl)-(7H-pyrrolo[2,3-d]-pyrimidine-4-yl)aminé ;
(1H-indazole-5-yl)-(7H-pyrrolo[2,3-d]pyrimidine-4-yl)-amine ;
chlorhydrate de 1-(4-m-tolylaminopyrrolo[2,3-d]-pyrimidine-7-yl)éthanone ;
(5-iodo-7H-pyrrolo[2,3-d]pyrimidine-4-yl)-m-tolylamine ;
4-(6-chloro-2,3-dihydro-indole-1-yl)-5H-pyrrolo-[3,2-d]pyrimidine-6-ol;
(3-éthynylphényl)-[7-(2-méthoxyéthyl)-7H-pyrrolo-[2,3-d]pyrimidine-4-yl]amine ;
(3-éthynylphényl)-{7-[2-(2-méthoxyéthoxy)éthyl]-7H-pyrrolo[2,3-d]pyrimidine-4-yl}amine ;
chlorhydrate de (7-allylpyrrolo[2,3-d]pyrimidine-4-yl)-(3-éthynylphényl)amine ;
chlorhydrate de (3-éthynylphényl)-(7-méthyl-pyrrolo[2,3-d]pyrimidine-4-yl)amine ;
(5-bromo-7H-pyrrolo[2,3-d]pyrimidine-4-yl)-(3-éthynylphényl)amine ;
(3-éthynylphényl)-(5-iodo-7H-pyrrolo[2,3-d]pyrimidine-4-yl)amine ;
acide 4-(3-éthynylphénylamino)-7H-pyrrolo[2,3-d]-pyrimidine-5-carboxylique ;
chlorhydrate de (3-éthynylphényl)-(5-méthyl-7H-pyrrolo[2,3-d]-pyrimidine-4-yl)amine ;
N-(5-iodo-7H-pyrrolo[2,3-d]pyrimidine-4-yl)-N-m-tolylacétamide ;
chlorhydrate d'ester méthylique de 4-(3-éthynylphénylamino)-7H-pyrrolo[2,3-d]pyrimidine-5-carboxylique ;
(3-éthynylphényl)-(5-méthylsulfanyl-7H-pyrrolo[2,3-d]-pyrimidine-4-yl)amine ; et
(3-éthynylphénylamino)-(7H-pyrrolo[2,3-d]pyrimidine-5-yl)carbonitrile.

12. Composé suivant la revendication 4, choisi dans le groupe consistant en
(3-éthynylphényl)-(7H-pyrrolo[2,3-d]pyrimidine-4-yl)-amine ;
(3-chlorophényl)-(7H-pyrrolo[2,3-d]pyrimidine-4-yl)-amine ;
(3-éthynylphényl)-(5-méthyl-7H-pyrrolo[2,3-d]-pyrimidine-4-yl)amine ;
ester méthylique d'acide 4-(3-éthynylphénylamino)-7H-pyrrolo[2,3-d]pyrimidine-5-carboxylique ;
4-(3-éthynylphénylamino)-7H-pyrrolo[2,3-d]pyrimidine-5-carbonitrile ;
(3-éthynylphényl)-(5-méthylsulfanyl-7H-pyrrolo[2,3-d]-pyrimidine-4-yl)amine ;
(1H-indole-5-yl)-(7H-pyrrolo[2,3-d]pyrimidine-4-yl)-amine ;
(5-bromo-7H-pyrrolo[2,3-d]pyrimidine-4-yl)-(3-éthynylphényl)amine ; et
(3-éthynylphénylamino)-(7H-pyrrolo[2,3-d]pyrimidine-5-yl)carbonitrile.

13. Composition pharmaceutique destinée au traitement d'un trouble hyperprolifératif chez un mammifère, qui comprend une quantité, apte au traitement d'une maladie hyperproliférative, d'un composé suivant la revendication 1 et un support pharmaceutiquement acceptable.

14. Composé suivant l'une quelconque des revendications 1 à 12, destiné à être utilisé en médecine.

15. Utilisation d'un composé suivant l'une quelconque des revendications 1 à 12 dans la production d'un médicament destiné au traitement d'un trouble hyperprolifératif.
